# EUROPEAN PATENT APPLICATION

(11) **EP 3 092 945 A1**
(43) Date of publication of application: **16.11.2016**
(21) Application number: 14876250.3
(22) Date of filing: 11.03.2014
(51) Int. Cl.: A61B 5/053

(54) **METHOD AND APPARATUS FOR MEASURING BODY FAT USING MOBILE DEVICE**

(30) Priority: 06.01.2014 KR 20140001504
(71) Applicant: Samsung Electronics Co., Ltd., Gyeonggi-do 16677 (KR)
(72) Inventor: KIM, Young-hyun, Suwon-si Gyeonggi-do 443-751 (KR); BAEK, Hyun-jae, Seoul 138-911 (KR); KIM, Kwang-choon, Suwon-si Gyeonggi-do 443-714 (KR); JO, Seong-wook, Suwon-si Gyeonggi-do 443-803 (KR)
(74) Representative: Gulde & Partner
(86) International application number: PCT/KR2014/002023
(87) International publication number: WO 2015/102156

(57) **Abstract**

A body fat measuring method of a mobile device includes inputting a predetermined intensity of current to an object via a first electrode in contact with a first surface of the object and a second electrode in contact with a second surface of the object, wherein the first and second electrodes are included in the mobile device; measuring a voltage between a third electrode and a fourth electrode that are included in the mobile device; acquiring impedance information of the object, based on the applied predetermined intensity of current and an intensity of the measured voltage; and acquiring body fat information of the object, based on the impedance information.

## Description

### TECHNICAL FIELD

The present invention relates to methods and apparatuses for measuring body fat, and more particularly, to a method and apparatus for accurately measuring body fat by using a mobile device.

### BACKGROUND ART

Body fat may be measured using various methods, such as, an underwater body density measuring method, a dual-energy x-ray absorptiometry (DEXA) method using X-rays, a subcutaneous fat measuring method using ultrasonic waves, a subcutaneous fat measuring method in which a thickness of a folded skin is measured, and a measuring method using reflection of infrared light. However, such methods require a long time and sufficient training to perform measurement, and measuring equipment for use in these methods are expensive, large, and difficult to manage.

A new body fat measuring method has been recently developed to address these problems, and body fat measuring equipment is also being developed to be compact and manageable. Various methods of accurately measuring body fat are also provided.

### DETAILED DESCRIPTION OF THE INVENTION

### TECHNICAL PROBLEM

The present invention provides a method and apparatus for measuring body fat.

### TECHNICAL SOLUTION

According to an aspect of the present invention, there is provided an impedance and body fat measuring method using a mobile device.

### ADVANTAGEOUS EFFECTS

According to an embodiment of the present invention, there is provided a method of measuring body fat of an object in a mobile device.

### BRIEF DESCRIPTION OF THE DRAWINGS

FIG. 1 illustrates an electrode arrangement of a mobile device according to an embodiment of the present invention.
FIG. 2 illustrates an example of an electrode arrangement of a mobile device according to an embodiment of the present invention.
FIG. 3 illustrates an example of an electrode arrangement of a mobile device according to an embodiment of the present invention.
FIG. 4 illustrates an example of an electrode arrangement of a mobile device according to an embodiment of the present invention.
FIGS. 5 and 6 are views for explaining the types of electrodes of a mobile device according to an embodiment of the present invention.
FIG. 7 illustrates a posture for measuring body fat by using a mobile device, according to an embodiment of the present invention.
FIG. 8 is a flowchart of a body fat measuring method using a mobile device, according to an embodiment of the present invention.
FIG. 9 is a block diagram of a mobile device according to an embodiment of the present invention.
FIG. 10 is a flowchart of a method of checking a contact state by applying a current to an object, according to an embodiment of the present invention.
FIG. 11 shows a change cycle of acquired impedance information, according to an embodiment of the present invention.
FIG. 12 is a flowchart of a method of checking a contact state based on an impedance information change cycle, according to an embodiment of the present invention.
FIG. 13 is a flowchart of a method of checking a contact state based on a reflected light value, according to an embodiment of the present invention.
FIG. 14 is a flowchart of a method of checking a contact state based on a pressure intensity, according to an embodiment of the present invention.
FIG. 15 is a flowchart of a method of determining whether a posture of an object needs to be corrected, based on an inclination variation, according to an embodiment of the present invention.
FIG. 16 is a flowchart of a method of determining whether a posture of an object needs to be corrected, based on an altitude variation, according to an embodiment of the present invention.
FIG. 17 is a flowchart of a method of determining whether a posture of an object needs to be corrected, based on a variation in impedance information, according to an embodiment of the present invention.
FIG. 18 is a flowchart of a method of determining whether a posture of an object needs to be corrected, based on a distance variation, according to an embodiment of the present invention.
FIG. 19 is a flowchart of a method of determining whether an action of an object is necessary, based on at least one of humidity information and temperature information, according to another embodiment of the present invention.
FIG. 20 is a flowchart of a method of determining an impedance correction value according to a posture of an object, according to an embodiment of the present invention.
FIG. 21 is a detailed flowchart of a method of determining an impedance correction value according to a posture of an object, according to an embodiment of the present invention.
FIG. 22 is a flowchart of a method of determining an impedance correction value according to an internal temperature of the mobile device, according to an embodiment of the present invention.
FIG. 23 is a flowchart of a method of determining an impedance correction value according to an internal temperature of the mobile device, according to an embodiment of the present invention.
FIGS. 24 and 25 illustrate postures of an object of which body fat is to be measured.
FIG. 26 is a flowchart of a method of providing information regarding a body fat measurement result of a mobile device, according to an embodiment of the present invention.
FIG. 27 is a flowchart of a method of providing information regarding a body fat measurement result of a mobile device, according to an embodiment of the present invention.
FIG. 28 is a block diagram of a mobile device according to an embodiment of the present invention.

### BEST MODE

According to an embodiment of the present invention, a body fat measuring method of a mobile device may include inputting a predetermined intensity of current to an object via a first electrode in contact with a first surface of the object and a second electrode in contact with a second surface of the object, wherein the first and second electrodes are included in the mobile device; measuring a voltage between a third electrode and a fourth electrode that are included in the mobile device; acquiring impedance information of the object, based on the applied predetermined intensity of current and an intensity of the measured voltage; and acquiring body fat information of the object, based on the impedance information.

The third and fourth electrodes may include contact type electrodes.

The third and fourth electrodes may include non-contact type electrodes.

The method may further include inputting a predetermined intensity of current to the first surface of the object via the first electrode; measuring the intensity of a current received by the third electrode via the first surface of the object; comparing the intensity of the current applied to the object via the first electrode with the measured intensity of the current received by the third electrode; and outputting a notification message including contact correction information based on a result of the comparison.

The method may further include acquiring change cycle information of the impedance information; comparing the acquired change cycle information with reference change cycle information; and outputting a notification message including contact correction information based on a result of the comparison.

The method may further include measuring a reflected light value of the first surface or the second surface of the object by using an optical sensor included in the mobile device; and outputting a notification message including contact correction information based on the measured reflected light value.

The method may further include measuring an intensity of pressure applied to the electrode by using a pressure sensor included in the mobile device; and outputting a notification message including contact correction information, based on the measured pressure intensity.

The method may further include measuring an inclination between the mobile device and the ground by using an acceleration sensor included in the mobile device; and outputting a notification message including posture correction information based on a variation in the measured inclination.

The method may further include measuring an altitude by using an altimeter included in the mobile device; and outputting a notification message including posture correction information based on a variation in the measured altitude.

The method may further include acquiring variation information of the acquired impedance information; comparing the acquired variation information with reference variation information; and outputting a notification message including posture correction information based on a result of the comparison.

The method may further include measuring a distance from a predetermined location by using an audio input unit and an audio output unit included in the mobile device; and outputting a notification message including posture correction information based on a variation in the measured distance.

The method may further include acquiring at least one of humidity information and temperature information by using at least one of a humidity sensor and a temperature sensor included in the mobile device; and outputting a notification message including action information, based on the acquired at least one of the humidity information and the temperature information.

The acquiring of the body fat information may include acquiring sensor measurement information from at least one of a magnetic sensor, an acceleration sensor, an altimeter, and a gyroscope sensor; estimating a posture of the object, based on the acquired sensor measurement information; determining an impedance correction value, based on the estimated posture of the object; and acquiring the body fat information, based on the acquired impedance information and the determined impedance correction value.

The acquiring of the body fat information may include measuring an internal temperature of the mobile device; determining an impedance correction value, based on the measured internal temperature; and acquiring the body fat information, based on the acquired impedance information and the determined impedance correction value.

According to an embodiment of the present invention, a method of providing information about a result of measuring body fat by a mobile device may include acquiring impedance information of an object, wherein the acquiring is performed by the mobile device; acquiring location information of the mobile device and determining whether the location information of the mobile device is equal to or greater than a critical value; and providing different notification messages to the object by reflecting the impedance information and a result of the determining of whether the location information of the mobile device is equal to or greater than the critical value.

The acquiring of the location information of the mobile device and the determining of whether the location information of the mobile device is equal to or greater than the critical value may include measuring an inclination between the mobile device and ground by using an acceleration sensor of the mobile device; and determining whether the measured inclination or a variation in the measured inclination is equal to or greater than a predetermined value.

The acquiring of the location information of the mobile device and the determining of whether the location information of the mobile device is equal to or greater than the critical value may include measuring an altitude of the mobile device by using an altimeter of the mobile device; and determining whether the measured altitude or a variation in the measured altitude is equal to or greater than a predetermined value.

The acquiring of the location information of the mobile device and the determining of whether the location information of the mobile device is equal to or greater than the critical value may include measuring a distance from a predetermined location by using an audio input unit and an audio output unit included in the mobile device; and determining whether a variation in the measured distance is equal to or greater than a predetermined reference value.

The location information of the mobile device may be determined based on sensor measurement information. The method may further include estimating a posture of the object; determining an impedance correction value, based on the estimated posture; and acquiring corrected impedance information based on the acquired impedance information and the impedance correction value. The providing of the different notification messages to the object by reflecting the impedance information and the result of the determining of whether the location information of the mobile device is equal to or greater than the critical value may include providing a notification message including posture correction information to the object, by reflecting the corrected impedance information and the result of the determining of whether the location information of the mobile device is equal to or greater than the critical value.

The method may further include measuring an internal temperature of the mobile device; determining an impedance correction value, based on the measured internal temperature; and acquiring corrected impedance information based on the acquired impedance information and the impedance correction value. The providing of the different notification messages to the object by reflecting the impedance information and the result of the determining of whether the location information of the mobile device is equal to or greater than the critical value may include providing a notification message including posture correction information to the object, by reflecting the corrected impedance information and the result of the determining of whether the location information of the mobile device is equal to or greater than the critical value.

The acquiring of the impedance information may include acquiring variation information of the impedance information; and comparing the variation information of the impedance information with reference variation information. The providing of the different notification messages to the object by reflecting the impedance information and the result of the determining of whether the location information of the mobile device is equal to or greater than the critical value may include providing a notification message including posture correction information to the object, by reflecting a result of the comparing and the result of the determining of whether the location information of the mobile device is equal to or greater than the critical value.

According to an embodiment of the present invention, a method of providing information about a result of measuring body fat by a mobile device may include acquiring impedance information of an object, wherein the acquiring is performed by the mobile device; acquiring information about a contact state of the mobile device and determining whether the information about the contact state of the mobile device is equal to or greater than a critical value; and providing different notification messages to the object by reflecting the information about the contact state of the mobile device and a result of the determining of whether the information about the contact state of the mobile device is equal to or greater than the critical value.

The acquiring of the information about the contact state of the mobile device and the determining of whether the information about the contact state of the mobile device is equal to or greater than the critical value may include measuring an intensity of pressure applied to the mobile device by using a pressure sensor of the mobile device; and determining whether the measured pressure intensity or a variation in the measured pressure intensity is equal to or greater than a predetermined reference value.

The acquiring of the information about the contact state of the mobile device and the determining of whether the information about the contact state of the mobile device is equal to or greater than the critical value may include measuring a reflected light value of at least one of a first surface and a second surface of the object by using an optical sensor of the mobile device; and determining whether the measured reflected light value or a variation in the measured reflected light value is equal to or greater than a predetermined value.

The acquiring of the information about the contact state of the mobile device and the determining of whether the information about the contact state of the mobile device is equal to or greater than the critical value may include applying a current to the object by using an electrode included in the mobile device; measuring an intensity of current received from the object via the electrode included in the mobile device; comparing the measured intensity of the current received from the object with the intensity of the current applied to the object via the electrode; and determining whether a difference between the two current intensities is equal to or greater than a predetermined value.

The method may further include measuring an internal temperature of the mobile device; determining an impedance correction value, based on the measured internal temperature; and acquiring corrected impedance information based on the acquired impedance information and the impedance correction value. The providing of the different notification messages to the object by reflecting the impedance information and the result of the determining of whether the information about the contact state of the mobile device is equal to or greater than the critical value may include providing a notification message including contact correction information to the object, by reflecting the corrected impedance information and the result of the determining of whether the information about the contact state of the mobile device is equal to or greater than the critical value.

The acquiring of the impedance information may include acquiring change cycle information of the impedance information; and comparing the change cycle information of the impedance information with reference change cycle information. The providing of the different notification messages to the object by reflecting the impedance information and the result of the determining of whether the information about the contact state of the mobile device is equal to or greater than the critical value may include providing a notification message including contact correction information to the object, by reflecting a result of the comparison and the result of the determining of whether the information about the contact state of the mobile device is equal to or greater than the critical value.

The method may further include acquiring at least one of humidity information and temperature information by using at least one of a humidity sensor and a temperature sensor included in the mobile device. The providing of the different notification messages to the object by reflecting the impedance information and the result of the determining of whether the information about the contact state of the mobile device is equal to or greater than the critical value may include providing a notification message including action information to the object, by reflecting the at least one of the humidity information and the temperature information, the impedance information, and the result of the determining of whether the information about the contact state of the mobile device is equal to or greater than the critical value.

According to an embodiment of the present invention, a non-transitory computer-readable recording medium has recorded thereon a computer program, which, when executed by a computer, performs the method.

According to an embodiment of the present invention, a mobile device may include a power unit that inputs a predetermined intensity of current to an object via a first electrode in contact with a first surface of the object and a second electrode in contact with a second surface of the object; a voltage measurer that measures a voltage between a third electrode and a fourth electrode that are included in the mobile device; a controller that acquires impedance information of the object, based on the applied predetermined intensity of current and an intensity of the measured voltage, and acquires body fat information of the object, based on the acquired impedance information; and an output unit that outputs the acquired impedance information and the acquired body fat information.

The third and fourth electrodes may include contact type electrodes.

The third and fourth electrodes may include non-contact type electrodes.

The power unit may input a predetermined intensity of current to the first surface of the object via the first electrode. The controller may measure the intensity of a current received by the third electrode via the first surface of the object, may compare the intensity of the current applied to the object via the first electrode with the measured intensity of the current received by the third electrode, and may control the output unit to output a notification message including contact correction information based on a result of the comparison.

The controller may acquire change cycle information of the impedance information, may compare the acquired change cycle information with reference change cycle information, and may control the output unit to output a notification message including contact correction information based on a result of the comparison.

The mobile device may further include at least one optical sensor, and the controller may measure a reflected light value of the first surface or the second surface of the object based on the at least one optical sensor and may control the output unit to output a notification message including contact correction information based on the measured reflected light value.

The mobile device may further include at least one pressure sensor, and the controller may measure an intensity of pressure applied to the electrode by using the at least one pressure sensor, and may control the output unit to output a notification message including contact correction information, based on the measured pressure intensity.

The mobile device may further include an acceleration sensor, and the controller may measure an inclination with respect to the ground by using the acceleration sensor, and may control the output unit to output a notification message including posture correction information based on a variation in the measured inclination.

The mobile device may further include an altitude sensor, and the controller may measure an altitude by using the altitude sensor and may control the output unit to output a notification message including posture correction information based on a variation in the measured altitude.

The controller may acquire variation information of the acquired impedance information, may compare the acquired variation information with reference variation information, and may control the output unit to output a notification message including posture correction information based on a result of the comparison.

The mobile device may further include an audio input unit and an audio output unit, and the controller may measure a distance from a predetermined location altitude by using the audio input unit and the audio output unit and may control the output unit to output a notification message including posture correction information based on a variation in the measured distance.

The mobile device may further include at least one of a humidity sensor and a temperature sensor, and the controller may acquire at least one of humidity information and temperature information by using the at least one of the humidity sensor and the temperature sensor, and may control the output unit to output a notification message including action information, based on the acquired at least one of the humidity information and the temperature information.

The mobile device may further include at least one of a magnetic sensor, an acceleration sensor, an altimeter, and a gyroscope sensor. The controller may further include a posture estimator that acquires sensor measurement information from the at least one sensor and estimates the posture of the object, based on the acquired sensor measurement information, and a correction value determiner that determines an impedance correction value according to the estimated posture of the object. The controller may acquire the body fat information, based on the acquired impedance information and the determined impedance correction value.

The controller may further include a correction value determiner that measures an internal temperature of the mobile device and determines an impedance correction value based on the measured internal temperature. The controller may acquire the body fat information, based on the acquired impedance information and the determined impedance correction value.

According to an embodiment of the present invention, a mobile device may include a controller configured to acquire impedance information of an object, acquire location information of the mobile device, and determine whether the location information of the mobile device is equal to or greater than a critical value; and an output unit configured to provide different notification messages to the object by reflecting the impedance information and a result of the determining of whether the location information of the mobile device is equal to or greater than the critical value.

The mobile device may further include a sensor unit configured to measure an inclination between the mobile device and ground by using an acceleration sensor of the mobile device, and the controller may determine whether the inclination measured by the sensor unit or a variation in the measured inclination is equal to or greater than a predetermined value.

The mobile device may further include a sensor unit configured to measure an altitude of the mobile device by using an altimeter of the mobile device, and the controller may determine whether the altitude measured by the sensor unit or a variation in the measured altitude is equal to or greater than a predetermined value.

The mobile device may further include an audio input unit and an audio output unit, and the controller may measure a distance from a predetermined location by using the audio input unit and the audio output unit and may determine whether a variation in the measured distance is equal to or greater than a predetermined value.

The mobile device may further include a sensor unit configured to acquire sensor measurement information. The location information of the mobile device may be determined based on the sensor measurement information. The controller may include a posture estimator configured to estimate a posture of the object, based on the acquired location information of the mobile device; and a correction value determiner configured to determine an impedance correction value based on the estimated posture, and may acquire corrected impedance information based on the acquired impedance information and the determined impedance correction value. The output unit may provide a notification message including posture correction information to the object, by reflecting the corrected impedance information and a result of the determining of whether the location information of the mobile device is equal to or greater than the critical value.

The mobile device may further include a sensor unit configured to measure an internal temperature of the mobile device. The controller may further include a correction value determiner configured to determine an impedance correction value based on the measured internal temperature. The controller may acquire corrected impedance information based on the acquired impedance information and the determined impedance correction value. The output unit may provide a notification message including posture correction information to the object, by reflecting the corrected impedance information and a result of the determining of whether the location information of the mobile device is equal to or greater than the critical value.

The controller may acquire variation information of the impedance information and compare the acquired variation information of the impedance information with reference variation information. The output unit may provide a notification message including posture correction information to the object, by reflecting a result of the comparison and the result of the determining of whether the location information of the mobile device is equal to or greater than the critical value.

According to an embodiment of the present invention, a mobile device may include a controller configured to acquire impedance information of an object, acquire information about a contact state of the mobile device, and determine whether the information about the contact state of the mobile device is equal to or greater than a critical value; and an output unit configured to provide different notification messages to the object by reflecting the impedance information and a result of the determining of whether the information about the contact state of the mobile device is equal to or greater than the critical value.

The mobile device may further include a sensor unit configured to measure an intensity of pressure applied to the mobile device by using a pressure sensor of the mobile device, and the controller may determine whether the pressure intensity measured by the sensor unit or a variation in the measured pressure intensity is equal to or greater than a predetermined value.

The mobile device may further include a sensor unit configured to measure a reflected light value of at least one of a first surface and a second surface of the object by using an optical sensor of the mobile device, and the controller may determine whether the reflected light value measured by the sensor unit or a variation in the measured reflected light value is equal to or greater than a predetermined value.

The controller may apply a current to a body of the object by using an electrode included in the mobile device, may measure an intensity of a current received from the object, may compare the measured intensity of the current received from the object with the intensity of the current applied via the electrode, and may determine whether a difference between the two current intensities is equal to or greater than a predetermined value.

The mobile device may further include a sensor unit configured to measure an internal temperature of the mobile device. The controller may further include a correction value determiner configured to determine an impedance correction value based on the measured internal temperature. The controller may acquire corrected impedance information based on the acquired impedance information and the determined impedance correction value. The output unit may provide a notification message including contact correction information to the object, by reflecting the corrected impedance information and a result of the determining of whether the information about the contact state of the mobile device is equal to or greater than the critical value.

The controller may acquire change cycle information of the impedance information and may compare the acquired change cycle information of the impedance information with reference change cycle information. The output unit may provide a notification message including contact correction information to the object, by reflecting a result of the comparing and the result of the determining of whether the information about the contact state of the mobile device is equal to or greater than the critical value.

### MODE OF THE INVENTION

Although general terms widely used at present were selected for describing the present invention in consideration of the functions thereof, these general terms may vary according to intentions of one of ordinary skill in the art, case precedents, the advent of new technologies, and the like. Terms arbitrarily selected by the applicant of the present invention may also be used in a specific case. In this case, their meanings need to be given in the detailed description of the present invention. Hence, the terms must be defined based on their meanings and the contents of the entire specification, not by simply stating the terms.

Throughout the specification, when an element is referred to as being "connected" or "coupled" to another element, it can be directly connected or coupled to the other element, or can be electrically connected or coupled to the other element with intervening elements interposed therebetween. In addition, the terms "comprises" and/or "comprising" or "includes" and/or "including" when used in this specification, specify the presence of stated elements, but do not preclude the presence or addition of one or more other elements. The terms "...unit" and "... module" when used in this specification refers to a unit in which at least one function or In operation is performed, and may be implemented as hardware, software, or a combination of hardware and software.

Throughout the specification, a mobile device may include not only a mobile device but also a case, a cover, and the like connected to or mounted on the mobile device. According to an embodiment of the present invention, examples of a mobile device may include, but are not limited to, portable apparatuses, such as a cellular phone, a smartphone, a tablet, a lap top computer, personal digital assistants (PDAs), a portable multimedia player (PMP), a navigation device, an MP3 player, and a digital camera, and may further include various other devices.

According to an embodiment of the present invention, examples of a case and a cover connected to or mounted on a mobile device include, but are not limited to, various shapes of covers and cases, such as a case capable of covering both front and rear sides of a cellular phone (e.g., a flip case), a case that covers only a rear side of a cellular phone, and a case that covers only an edge of a cellular phone.

Throughout the specification, impedance information may include bioimpedance and body fat, and body fat information may include all pieces of information, such as a body fat mass, a percent body fat, and a body water mass.

Throughout the specification, a notification message may refer to any method of notifying a user of a mobile device of information for use in body resistance measurement, such as a text message via a display, a voice message, a notification via vibration, lamp flickering, and a lamp color change.

Throughout the specification, an object may denote a user or the body of the user.

Embodiments of the present invention are described in detail herein with reference to the accompanying drawings so that this disclosure may be easily performed by one of ordinary skill in the art to which the present invention pertain. The invention may, however, be embodied in many different forms and should not be construed as being limited to the embodiments set forth herein. In the drawings, parts irrelevant to the description are omitted for simplicity of explanation, and like numbers refer to like elements throughout.

The present invention will now be described more fully with reference to the accompanying drawings, in which exemplary embodiments of the invention are shown.

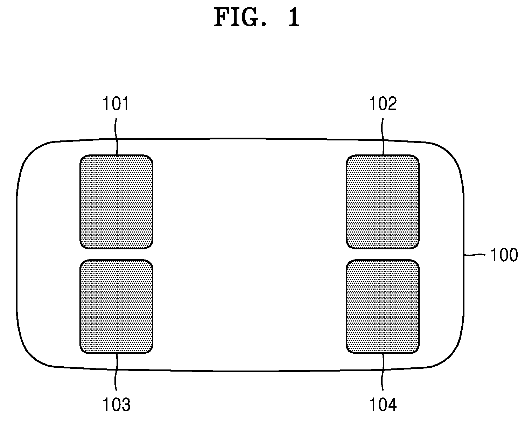

FIG. 1 illustrates an electrode arrangement of a mobile device according to an embodiment of the present invention.

According to an embodiment of the present invention, a mobile device 100 may be a device that measures body resistance and body fat. The mobile device 100 may measure body fat by using a bioimpedance method of measuring electrical impedance of an object by applying current to the object.

According to an embodiment of the present invention, the mobile device 100 may include four electrodes 101, 102, 103, and 104.

According to an embodiment of the present invention, the first electrode 101 may contact a first surface of the object, and the second electrode 102 may contact a second surface of the object. According to an embodiment of the present invention, the first surface of the object may denote a left hand of the object, the second surface of the object may denote a right hand of the object, and the object may denote the body of a user.

According to an embodiment of the present invention, the mobile device 100 may apply a predetermined magnitude of current by using the first and second electrodes 101 and 102. For example, the a predetermined magnitude of current may be an alternating current (AC), which may have a frequency of 50 KHz to 1000 KHz. The mobile device 100 may sequentially apply ACs having various frequencies. A predetermined magnitude of current applied via the first electrode 101 may be input to the second electrode 102 via the first surface of the object and the second surface of the object.

According to an embodiment of the present invention, the mobile device 100 may measure a voltage between the third and fourth electrodes 103 and 104. In other words, the mobile device 100 may measure a voltage of the object via the third and fourth electrodes 103 and 104. According to an embodiment of the present invention, the third and fourth electrodes 103 and 104 may be contact type electrodes or non-contact type electrodes.

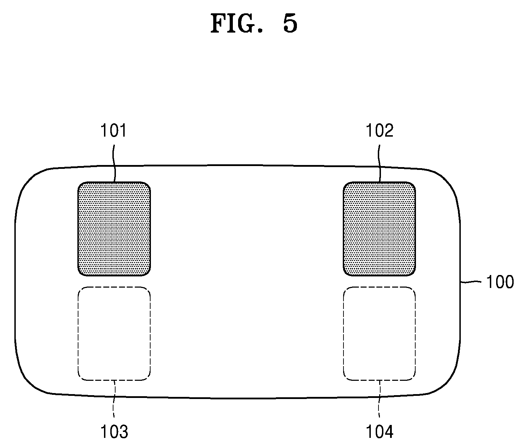

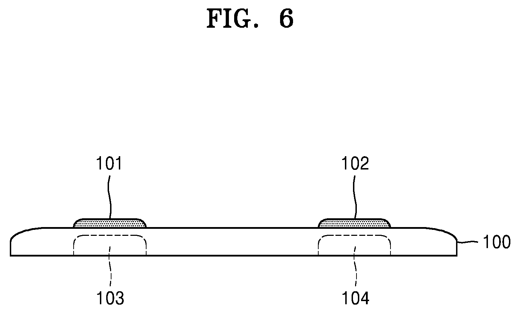

According to an embodiment of the present invention, a contact type electrode denotes an electrode that is used to measure a voltage while in contact with an object. In other words, the third electrode 103 and the fourth electrode 104 may contact the first surface and the second surface of the object, respectively, and the mobile device 100 may measure the voltage between the third electrode 103 and the fourth electrode 104. In other words, the mobile device 100 may measure a voltage applied to the object via the third and fourth electrodes 103 and 104. A non-contact type electrode will be described later with reference to FIGS. 5 and 6.

According to an embodiment of the present invention, the mobile device 1000 may measure impedance of the object. In other words, the mobile device 100 may measure impedance of the object, based on the predetermined magnitude of current applied via the first electrode 101 and the second electrode 102 and the measured voltage between the third electrode 103 and the fourth electrode 104.

According to an embodiment of the present invention, the mobile device 1000 may include a reference resistor. The reference resistor may denote a resistor having a predetermined value. According to an embodiment of the present invention, the mobile device 100 may apply the predetermined magnitude of current having been applied to the object via the first and second electrodes 101 and 102 to the reference resistor and may measure a voltage between both ends of the reference resistor. The mobile device 100 may more accurately measure the impedance of the object, based on the voltage between both ends of the reference resistor and the measured voltage between the third and fourth electrodes 103 and 104.

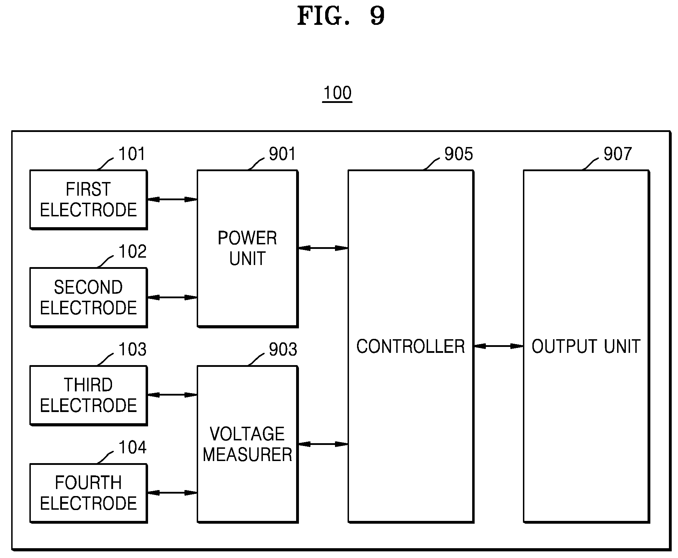

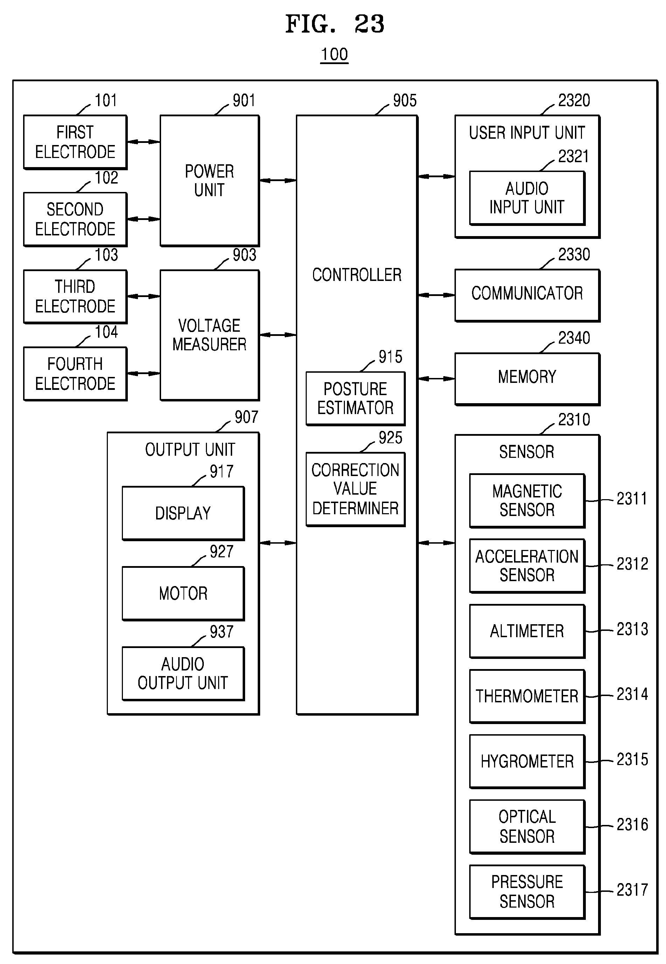

According to an embodiment of the present invention, the mobile device 100 may include only two electrodes, and may apply a current to the object by using the two electrodes and may measure a voltage. Additional components of the mobile device 100 will be described in detail later with reference to FIGS. 9 and 23.

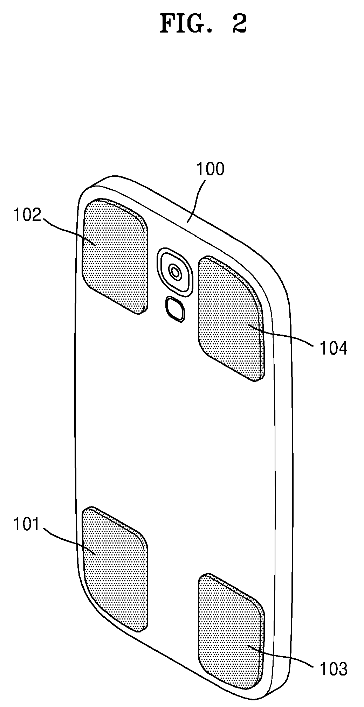

FIG. 2 illustrates an example of an electrode arrangement of a mobile device according to an embodiment of the present invention.

According to an embodiment of the present invention, electrodes included in the mobile device may be arranged in various shapes. In other words, according to an embodiment of the present invention, the electrodes included in the mobile device may be arranged in various shapes by taking into account user convenience, a design, and the shape and structure of the mobile device.

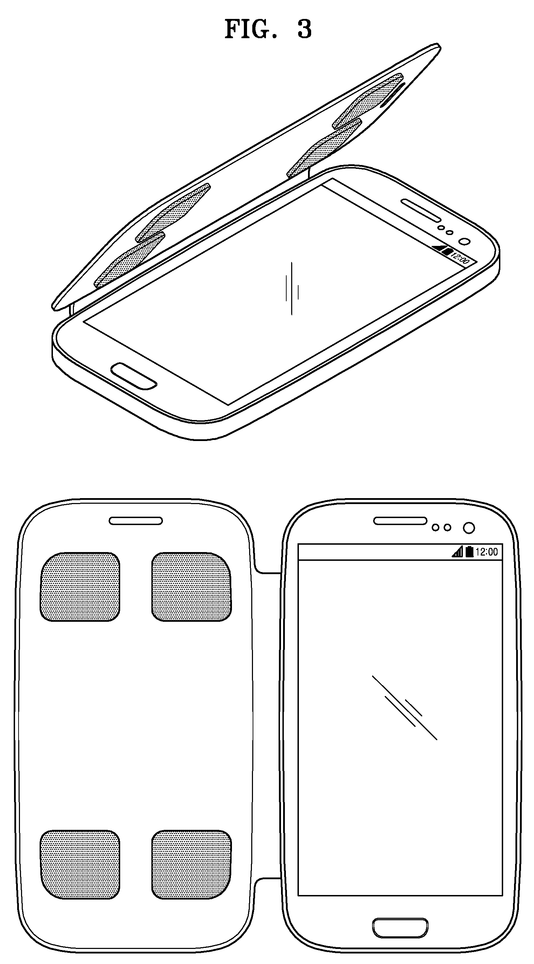

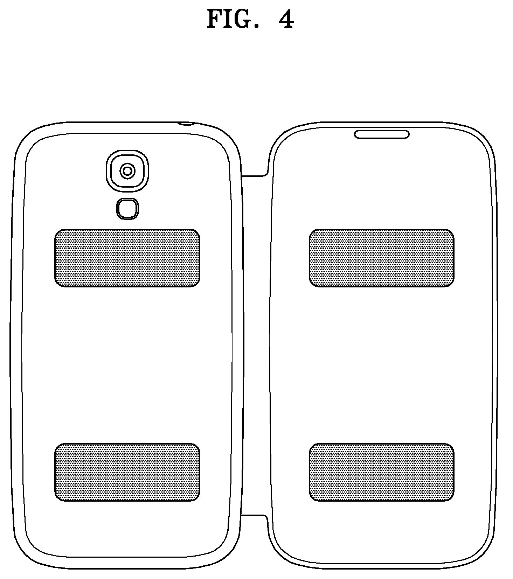

For example, in the mobile device 100 of FIG. 2, the first electrode 101, the second electrode 102, the third electrode 103, and the fourth electrode 104 are arranged as close as possible to the corners of the mobile device 100. The second electrode and the fourth electrode may be arranged as being connected to each other in an area except for a camera, and electrodes may be arranged on a flip cover of a mobile device instead of on a rear surface of the mobile device. Other embodiments related to an electrode arrangement will be described later with reference to FIGS. 3 and 4. The electrodes may also be arranged by avoiding an area on the mobile device 100 where an antenna is disposed, so that transmission and reception of electric waves are not interrupted.

According to an embodiment of the present invention, a logo inscribed on the mobile device 100 may be used as an electrode, and an edge of the mobile device 100 may be processed as an electrode, whereby user convenience may improve since measurement may be achieved whenever a user holds any portion of the mobile device 100.

Moreover, according to an embodiment of the present invention, electrodes included in the mobile device 100 may be formed in various shapes.

Although 4 electrodes are rectangular in FIG. 2, the 4 electrodes may be circular, trapezoidal, or triangular, and the 4 electrodes may respectively have 4 different shapes. By enlarging the electrodes and narrowing the space between the electrodes, the electrodes may be arranged as the pattern of the mobile device. The first electrode 101, the second electrode 102, the third electrode 103, and the fourth electrode 104 may be arranged asymmetrically.

The electrodes of the mobile device may be arranged to be higher than the surface of the mobile device so that a user may easily grab the electrodes, or the electrodes may be inlaid via a dot pattern, a hair pattern, engraving, or the like so that a user easily ascertains the locations of the electrodes, or the case of the mobile device may be made convex in order to prevent the electrodes from being scratched.

An auxiliary display may be on the rear side of the mobile device so that a user may read a message or information via the auxiliary display.

According to an embodiment of the present invention, the electrodes may be formed of any of various materials. For example, conductive rubber, plastic, fabric, and a ceramic material may be used to form the electrodes, and metal may also be used. When metal is used, the metal may be coated with TiN, TiCN, CrN, or the like in order to increase surface conductivity of the metal and scratch resistance of an electrode surface.

FIG. 3 illustrates an example of an electrode arrangement of a mobile device according to an embodiment of the present invention.

Referring to FIG. 3, first through fourth electrodes are arranged on a cover of the mobile device. In other words, as shown in FIG. 3, some or all of the components of the mobile device necessary for measuring body fat, including the first through fourth electrodes, may be included in the case of the mobile device.

As described above, a mobile device throughout the specification may denote the case of the mobile device. In other words, according to an embodiment of the present invention, a case for mobile devices may be mounted on a mobile device, and body fat may be measured using components included in the case of the mobile device.

According to an embodiment of the present invention, the shape of an electrode arrangement on the case of the mobile device is not limited. As shown in FIG. 3, the first through fourth electrodes may be arranged in an engraved structure in order to prevent contact with a display of the mobile device. As shown in FIG. 2, the first through fourth electrodes may be arranged to be at a raised level so that an object may more easily contact the electrodes. The first through fourth electrodes may be included in the mobile device, the cover of the mobile device, or narrow lateral sides of the case of the mobile device.

As described above, the shapes and materials of the electrodes included in the case of the mobile device are not limited.

FIG. 4 illustrates an example of an electrode arrangement of a mobile device according to an embodiment of the present invention.

Referring to FIG. 4, first through fourth electrodes are arranged on a mobile device and a case of the mobile device. In other words, components of the mobile device necessary for measuring body fat, including the first through fourth electrodes, may be included and distributed in the mobile device and the case of the mobile device.

As described above, a mobile device throughout the specification may also denote the case of a mobile device. In other words, a case for mobile devices may be mounted on a mobile device and the mobile device may measure body fat by using components included in the case of the mobile device.

A logo of a flip cover may be used instead of electrodes. In other words, the logo of the flip cover may be formed of the same material used to form the electrodes, and thus may be used as at least one of the first through fourth electrodes. As described above, the first through fourth electrodes included in the mobile device and the case of the mobile device are not limited in terms of arrangement, shape, and material.

FIGS. 5 and 6 are views for explaining the types of electrodes of a mobile device according to an embodiment of the present invention.

FIG. 5 explains a non-contact type electrode. In other words, the third electrode 103 and the fourth electrode 104 do not need to always contact the first surface or the second surface of the object.

In other words, according to an embodiment of the present invention, since ACs are applied to the first and second electrodes 101 and 102, the mobile device 100 may measure an AC voltage between the third electrode 103 and the fourth electrode 104 when the third electrode 103 and the fourth electrode 104 are not in contact with the object, based on mutual inductance and a capacitive coupling effect. For example, a non-contact type electrode may be constructed via a sensor for measuring a change in a magnetic field in a non-contact state, such as an epic sensor. By measuring a change in a magnetic field due to application of AC, the mobile device 100 may measure a voltage between the first surface of the object and the second surface thereof. The capacitive coupling effect and the epic sensor are obvious to one of ordinary skill in the art, and thus detailed descriptions thereof will be omitted.

According to an embodiment of the present invention, when the third electrode 103 and the fourth electrode 104 are non-contact type electrodes, they do not need to contact the object and thus do not need to be disposed on a surface of the mobile device. In other words, the third electrode 103 and the fourth electrode 104 may be disposed within the mobile device or the cover or case of the mobile device. The third and fourth electrodes 103 and 104 may be included in narrow lateral sides of the mobile device or the cover or case of the mobile device.

In other words, referring to FIG. 6, the first electrode 101 and the second electrode 102 may be arranged outside the mobile device or the cover or case of the mobile device, and the third electrode 103 and the fourth electrode 104 indicated by a dotted line may be hidden under the case. As described above with reference to FIGS. 1- 4, the first and second electrodes 101 and 102, which are contact type electrodes, and the third and fourth electrodes 103 and 104, which are non-contact type electrodes, may be arranged using various methods.

According to an embodiment of the present invention, the third electrode and the fourth electrode are adjacent to the first surface and the second surface of the object, respectively. Being adjacent may mean that the third electrode 103 and the fourth electrode 104 are located within predetermined distances from the first surface and the second surface of the object, respectively.

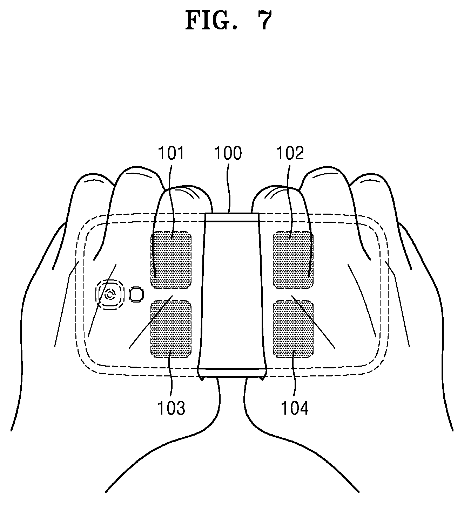

FIG. 7 illustrates a posture for measuring body fat by using a mobile device, according to an embodiment of the present invention.

Referring to FIG. 7, in the body fat measuring posture according to an embodiment of the present invention, an object grabs the mobile device 100 with both hands, the first electrode 101 and the third electrode 103 included in the mobile device 100 may contact the left hand, and the second electrode 102 and the fourth electrode 104 included in the mobile device 100 may contact the right hand.

The method of FIG. 7 is merely an example of a body fat measuring method, and body fat may be simply measured when the mobile device is put on both hands of the object. Alternatively, body fat may be measured when a body part (for example, a wrist or a finger) other than the hands contacts at least one electrode.

As described above, a method of arranging the first through fourth electrodes 101-104 is not limited, and, in particular, the third and fourth electrodes 103 and 104 do not need to contact the object in order to measure body fat of the object.

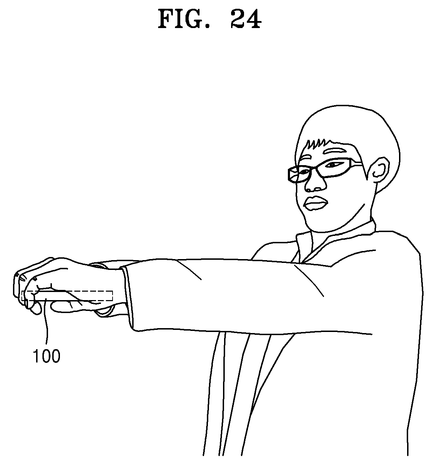

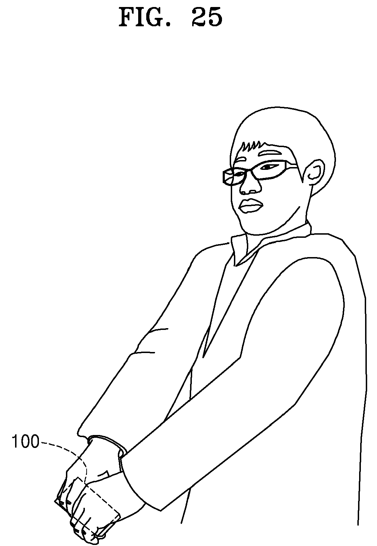

The posture of the object for measuring body fat of the object, according to an embodiment of the present invention, will be further described later with reference to FIGS. 24 and 25.

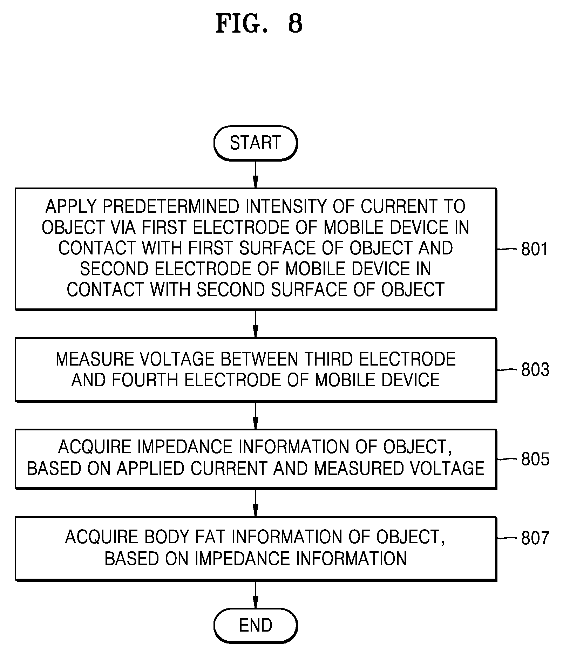

FIG. 8 is a flowchart of a body fat measuring method using a mobile device, according to an embodiment of the present invention.

In operation 801, the mobile device may apply a predetermined intensity of current to an object via a first electrode of the mobile device in contact with a first surface of the object and a second electrode of the mobile device in contact with a second surface of the object.

According to an embodiment of the present invention, the mobile device applies a predetermined intensity of current to the object in order to measure impedance of the object. The predetermined intensity of current may include AC having a predetermined intensity and AC having a predetermined frequency.

In operation 803, the mobile device may measure a voltage between a third electrode and a fourth electrode of the mobile device.

According to an embodiment of the present invention, the third electrode and the fourth electrode may be contact type electrodes or non-contact type electrodes. When the third electrode and the fourth electrode are contact type electrodes, the mobile device may measure the voltage between the third electrode and the fourth electrode when the third electrode and the fourth electrode are in contact with the first surface and the second surface of the object, respectively. When the third electrode and the fourth electrode are non-contact type electrodes, the mobile device may measure the voltage between the third electrode and the fourth electrode when the third electrode and the fourth electrode do not contact the first surface and the second surface of the object and are respectively close to the first surface and the second surface.

In operation 805, the mobile device may acquire impedance information of the object, based on the applied current and the measured voltage.

According to an embodiment of the present invention, the mobile device may measure body resistance, which is the impedance of the object, based on the current applied to the object via the first and second electrodes and the measured voltage between the third and fourth electrodes, which is a voltage applied to the object.

According to an embodiment of the present invention, the mobile device may include a reference resistor, and the mobile device may apply the predetermined magnitude of current applied to the object via the first and second electrodes to the reference resistor, measure a voltage between both ends of the reference resistor, and compare the measured voltage between both ends of the reference resistor with the measured voltage between the third and fourth electrodes, thereby accurately measuring the impedance of the object.

According to an embodiment of the present invention, the mobile device may determine a contact state between the electrodes and the object in order to accurately measure the impedance of the object, and may selectively output a notification message including contact correction information, based on the contact state.

According to an embodiment of the present invention, the mobile device may apply a predetermined intensity of current to the first electrode and measure the intensity of a current received by the third electrode via the first surface of the object, thereby determining a contact state between the object and the mobile device and selectively outputting a notification message including contact correction information.

According to an embodiment of the present invention, the mobile device may acquire change cycle information of the impedance information and compare the acquired change cycle information with reference change cycle information, thereby determining a contact state between the first through fourth electrodes and the object and selectively outputting a notification message including contact correction information.

According to an embodiment of the present invention, the mobile device may measure a reflected light value by using an optical sensor included in the mobile device, may determine a contact state between the first through fourth electrodes and the object based on the measured reflected light value, and may selectively output a notification message including contact correction information.

According to an embodiment of the present invention, the mobile device may measure the intensity of pressure by using a pressure sensor included in the mobile device, may determine a contact state between the first through fourth electrodes and the object based on the measured pressure intensity, and may selectively output a notification message including contact correction information.

According to an embodiment of the present invention, the mobile device may output a notification message instructing the posture of the object to be corrected, in order to more accurately measure the impedance of the object.

According to an embodiment of the present invention, the mobile device may measure an inclination with respect to the ground by using an acceleration sensor included in the mobile device, and may selectively output a notification message including posture correction information, based on a variation in the measured inclination.

According to an embodiment of the present invention, the mobile device may measure an altitude by using an altimeter included in the mobile device, and may selectively output a notification message including posture correction information, based on a variation in the measured altitude.

According to an embodiment of the present invention, the mobile device may acquire variation information of the impedance information acquired by the mobile device, may compare the acquired variation information with reference variation information, and may selectively output a notification message including posture correction information.

According to an embodiment of the present invention, the mobile device may measure a distance from a predetermined location by using an audio input unit and an audio output unit included in the mobile device, and may selectively output a notification message including posture correction information, based on a variation in the measured distance.

According to an embodiment of the present invention, the mobile device may acquire at least one of humidity information and temperature information by using at least one of a humidity sensor and a temperature sensor included in the mobile device, and may selectively output a notification message including action information, based on the acquired at least one of the humidity information and the temperature information.

In operation 807, the mobile device may acquire body fat information of the object, based on the impedance information acquired in operation 805.

According to an embodiment of the present invention, the mobile device may acquire sensor measurement information from at least one sensor included in the mobile device, may estimate a posture of the object based on the acquired sensor measurement information to determine an impedance correction value, and may acquire the body fat information based on the impedance information acquired in operation 805 and the determined impedance correction value.

The mobile device may determine the impedance correction value according to an internal temperature of the mobile device, and may acquire the body fat information based on the impedance information acquired in operation 805 and the determined impedance correction value.

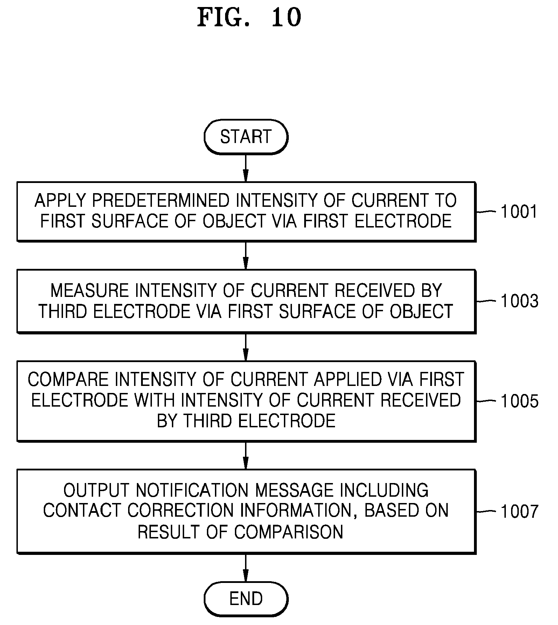

The outputting of a notification message according to a contact state and an action of the object, and the acquiring of the body fat information based on the impedance correction value, according to an embodiment of the present invention, will be described later in detail with reference to FIGS. 10-22.

FIG. 9 is a block diagram of a mobile device according to an embodiment of the present invention.

The mobile device 100 according to an embodiment of the present invention may include a first electrode 101, a second electrode 102, a third electrode 103, a fourth electrode 104, a power unit 901, a voltage measurer 903, a controller 905, and an output unit 907.

According to an embodiment of the present invention, the first electrode 101 contacts the first surface of the object, and the first surface of the object may denote the left hand of the object, the left hand fingers of the object, or a left side of the object.

According to an embodiment of the present invention, the second electrode 102 contacts the second surface of the object, and the second surface of the object may denote the right hand of the object, the right hand fingers of the object, or a right side of the object.

According to an embodiment of the present invention, the power unit 901 may input a current having a predetermined intensity to the object via the first and second electrodes 101 and 102.

According to an embodiment of the present invention, the power unit 901 may denote a battery included in a mobile device or an auxiliary power supply rather than the battery, but embodiments are not limited thereto. The power unit 901 may denote a component that applies a predetermined current to the object via the first electrode and the second electrode.

According to an embodiment of the present invention, the third electrode 103 may denote an electrode that contacts the first surface of the object or an electrode located adjacent to the first surface of the object within a predetermined distance from the first surface of the object. The fourth electrode 104 may denote an electrode that contacts the second surface of the object or an electrode located adjacent to the second surface of the object within a predetermined distance from the second surface of the object. In other words, as described above, the third electrode 103 and the fourth electrode 104 may be contact type electrodes or non-contact type electrodes.

According to an embodiment of the present invention, the power unit 901 may apply a predetermined current to the first surface of the object via the first electrode 101 and may measure the intensity of a current received by the third electrode 103 via the object. According to an embodiment of the present invention, the controller 905 may ascertain a contact state of the object by comparing the intensity of current applied to the first electrode 101 with the intensity of current received by the third electrode, and may control the output unit 907 to selectively output a notification message including contact correction information.

According to an embodiment of the present invention, the voltage measurer 903 may measure a voltage applied between the third and fourth electrodes 103 and 104. In other words, the power unit 901 may measure a voltage applied to the object via the third electrode 103 and the fourth electrode 104, based on the current applied to the object via the first electrode 101 and the second electrode 102.

According to an embodiment of the present invention, the controller 905 may acquire impedance information of the object based on the current applied by the power unit 901 and the voltage measured by the voltage measurer 903, and may measure body fat information based on measured impedance. The controller 905 may include a calculation unit of the mobile device, such as a central processing unit (CPU), and may acquire the impedance information and the body fat information of the object via a calculation of measured information.

According to an embodiment of the present invention, the controller 905 may change an analog signal measured to acquire the impedance information of the object to a digital signal.

According to an embodiment of the present invention, the controller 905 may acquire change cycle information of the acquired impedance information and compare the acquired change cycle information with reference change cycle information to ascertain a contact state between the object and each electrode, and may control the output unit 907 to selectively output a notification message including contact correction information.

According to an embodiment of the present invention, the controller 905 may measure a reflected light value of the first surface or the second surface of the object based on at least one optical sensor included in the mobile device 100, and may control, based on the measured reflected light value, the output unit 907 to output a notification message including contact correction information.

According to an embodiment of the present invention, the controller 905 may measure the intensity of pressure applied to at least one electrode based on at least one pressure sensor included in the mobile device 100, and may control, based on the measured pressure intensity, the output unit 907 to output a notification message including contact correction information.

According to an embodiment of the present invention, the controller 905 may measure an inclination of the mobile device 100 with respect to the ground by using an acceleration sensor included in the mobile device 100, and may control, based on a variation in the measured inclination, the output unit 907 to output a notification message including posture correction information.

According to an embodiment of the present invention, the controller 905 may measure an altitude of the mobile device 100 with respect to the ground by using an altimeter included in the mobile device 100, and may control, based on a variation in the altitude, the output unit 907 to output a notification message including posture correction information.

According to an embodiment of the present invention, the controller 905 may acquire variation information of impedance information, may compare the acquired variation information with reference variation information, and may control the output unit 907 to output a notification message including posture correction information.

According to an embodiment of the present invention, the controller 905 may measure a distance from a predetermined location by using an audio input unit (not shown) and an audio output unit (not shown) included in the mobile device 100, and may control, based on a variation in the measured distance, the output unit 907 to output a notification message including posture correction information.

According to an embodiment of the present invention, the controller 905 may acquire at least one of humidity information and temperature information by using at least one of a humidity sensor and a temperature sensor included in the mobile device 100, and may control the output unit 907 to output a notification message including action information, based on the acquired at least one of the humidity information and the temperature information.

According to an embodiment of the present invention, the controller 905 may acquire sensor measurement information from at least one sensor included in the mobile device 100, may estimate a posture of the object based on the acquired sensor measurement information, may determine an impedance correction value based on the estimated posture, and may acquire the body fat information of the object based on the determined impedance correction value and the acquired impedance information.

According to an embodiment of the present invention, the controller 905 may measure an internal temperature of the mobile device 100, may determine an impedance correction value based on the measured internal temperature, and may acquire the body fat information based on the determined impedance correction value and the acquired impedance information.

According to an embodiment of the present invention, the output unit 907 may output the impedance information and the body fat information, and may output a notification message including the contact correction information, the posture correction information, or the action information.

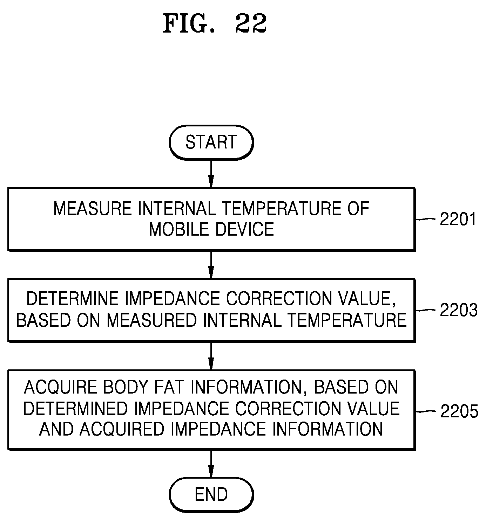

According to an embodiment of the present invention, the mobile device 100 may further include additional components, such as a communicator and a memory, which will be described later in detail with reference to FIG. 22.

FIG. 10 is a flowchart of a method of checking a contact state by applying a current to an object, according to an embodiment of the present invention.

In operation 1001, a mobile device may apply a predetermined intensity of current to the first surface of the object via the first electrode.

In operation 1003, the mobile device may measure the intensity of current received by the third electrode via the first surface of the object.

In operation 1005, the mobile device may compare the intensity of the current applied via the first electrode with the intensity of the current received by the third electrode.

In other words, in order to determine whether the first surface of the object contacts the first and third electrodes by at least a predetermined area, the mobile device may apply a predetermined intensity of current via the first electrode and measure the intensity of current received by the third electrode to thereby determine a contact state between the first surface of the object and the first and third electrodes.

According to an embodiment of the present invention, when the first surface of the object contacts the first and third electrodes by the predetermined area or larger, the intensity of current flowing on a skin surface of the object is greatly different from the intensity of the current applied via the first electrode, and thus the mobile device may compare the intensity of current applied to the first electrode with the intensity of current received by the third electrode to thereby determine a contact state between the object and the first and third electrodes.

In operation 1007, the mobile device may output a notification message including contact correction information, based on a result of the comparison.

For example, when the intensity of current applied to the first electrode is less than or equal to a predetermined current intensity, the mobile device may output a notification message instructing the object to check contact of the first surface of the object.

According to an embodiment of the present invention, the mobile device may output a notification message including contact correction information by determining whether a portion of the first surface of the object that contacts the first electrode is less than or equal to the predetermined area.

According to an embodiment of the present invention, the contact correction information includes information requesting the object to adjust the contact state of the object. For example, the mobile device may output a message instructing a user to position his or her left hand on an electrode. The contact correction information may further include whether the electrode poorly contacts the first surface or the second surface of the object.

According to an embodiment of the present invention, the notification message including the contact correction information may be output in the form of a voice message via a speaker, an image via a display, a text, lamp light, or a vibration. However, embodiments are not limited thereto, and the notification message including the contact correction information may be output in various other forms.

According to an embodiment of the present invention, each operation of FIG. 10 may be performed before each operation of FIG. 8 is performed. In other words, the mobile device may check a contact state within a short period of time before measuring body fat, thereby preventing re-measurement due to an abnormal contact state.

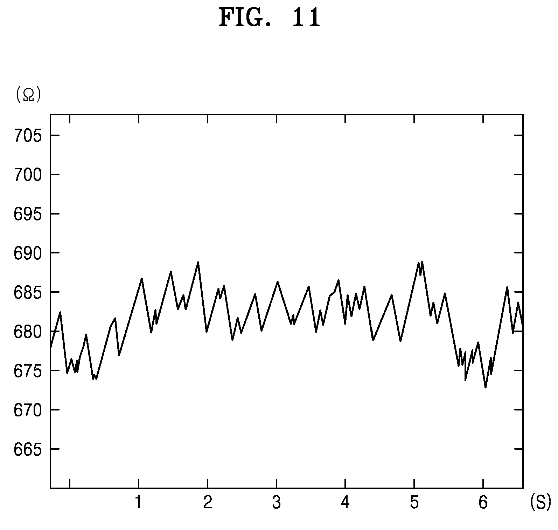

FIG. 11 shows a change cycle of acquired impedance information, according to an embodiment of the present invention.

FIG. 11 is a graph showing a variation in impedance of the object over time. In the graph of FIG. 11, a horizontal axis indicates time expressed in a unit of s, and the vertical axis indicates impedance expressed in a unit ofΩ.

According to an embodiment of the present invention, the impedance of the object varies depending on a pulse wave that is a blood flow due to a heart activity. For example, a heart rate is about 50 to 160 times per minute according to persons, and the impedance of the object may vary depending on a pulse wave that is a blood flow occurring due to heartbeat, as in the graph of FIG. 11. For example, the impedance of the object may change from 680 ohm (Ω) within a predetermined range according to a pulse wave, and may vary depending on a change cycle that is similar to heartbeat.

When the mobile device measures the impedance of the object, if the impedance does not vary depending on a pulse wave or the measured impedance has a too-high average value, the object and each electrode do not accurately contact each other, and thus the impedance of the object may not be properly measured. Accordingly, the mobile device may acquire change cycle information of the impedance of the object to determine a contact state between the object and each electrode, and may compare the acquired change cycle information with reference change cycle information.

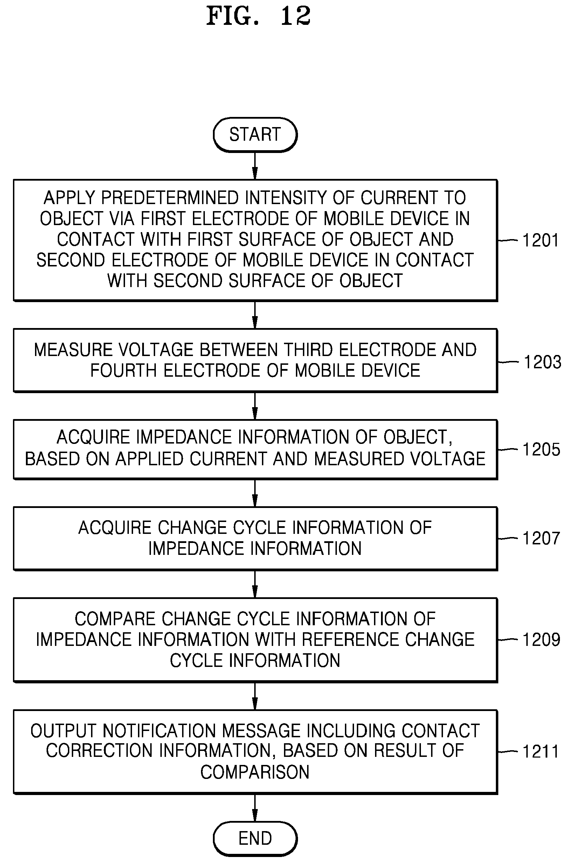

FIG. 12 is a flowchart of a method of checking a contact state based on an impedance information change cycle, according to an embodiment of the present invention.

Operations 1201-1205 correspond to operations S801-S805 of FIG. 8, respectively, and thus detailed descriptions thereof will be omitted.

In operation 1207, the mobile device may acquire change cycle information of the impedance information. In other words, the mobile device may acquire information about the change cycle according to a pulse wave of the impedance information described above with reference to FIG. 11.

In operation 1209, the mobile device may compare the change cycle information of the impedance information acquired in operation 1207 with reference change cycle information.

According to an embodiment of the present invention, the reference change cycle information may include impedance change cycle information determined according to a heart rate of the object or a statistical heart rate, and may include information about a fluctuation amount and an average body resistance (impedance). The reference change cycle information may be stored in the mobile device.

According to an embodiment of the present invention, the mobile device may measure a heart rate or a pulse wave of the object, and may acquire reference change cycle information based on the measured heart rate or pulse wave.

In operation 1211, the mobile device may output a notification message including contact correction information, based on a result of the comparison.

According to an embodiment of the present invention, the mobile device may compare the change cycle information acquired in operation 1207 with the reference change cycle information and thus may determine that impedance does not change or the measured impedance has a too-high average value or that a change cycle of the impedance is greatly different from the heart rate of the object, namely, the object and the electrode do not accurately contact each other and thus the impedance of the object is not properly measured. In this case, the mobile device may output a notification message including contact correction information in order to accurately measure the impedance of the object. When it is determined that the contact state is good, the mobile device may continuously measure body fat.

For example, the mobile device may output a message instructing the user to accurately position his or her right hand on each electrode. The mobile device may notify the object that a contact state between the object and electrodes included in the mobile device is not good, via a sound, vibration, or an image.

According to an embodiment of the present invention, in operations 1207 and 1209, the mobile device may determine the contact state between the object and each electrode by acquiring only change cycle information corresponding to a predetermined time period, before measuring the impedance of the object.

For example, assuming that 20 seconds are required to complete up to impedance measurement, the mobile device may acquire only change cycle information of impedance information corresponding to 5 seconds, and may determine the contact state according to the acquired change cycle information corresponding to 5 seconds.

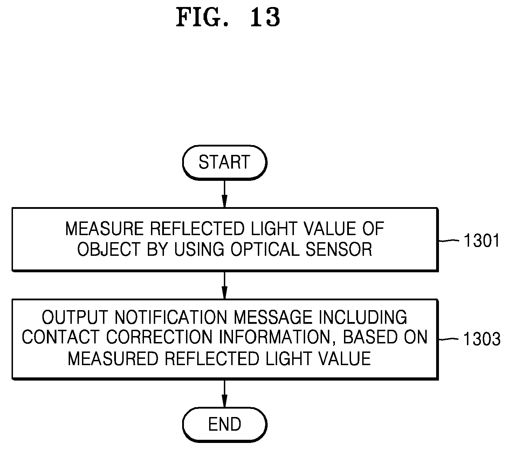

FIG. 13 is a flowchart of a method of checking a contact state based on a reflected light value, according to an embodiment of the present invention.

In operation 1301, the mobile device may measure a reflected light value of the object by using an optical sensor.

According to an embodiment of the present invention, the mobile device may include an optical sensor, which may include a light-emission device and a light-reception device. According to an embodiment of the present invention, the light-emission device may include a light source, such as an LED, and may be a light source that generates visible light (400 to 600 nm), near-infrared light (600 to 1200 nm), and infrared light (1200 to 2500 nm).

According to an embodiment of the present invention, the optical sensor may be included in at least one of the first through fourth electrodes included in the mobile device. In other words, the optical sensor may be included in each of the first electrode and the second electrode, and the light-emission device and the light-reception device may be separately included in at least one of the first through fourth electrodes.

The optical sensor may be included in only the first and third electrodes contacting the first surface of the object, or may be included in only one of the first and third electrodes. In other words, an arrangement of the optical sensor is not limited.

In operation 1303, the mobile device may output a notification message including contact correction information, based on the measured reflected light value.

For example, when the object and electrodes do not accurately contact each other, the intensity or value of light emitted from the light-emission device of the optical sensor that is reflected by the object and input to the light-reception device may be less than a predetermined intensity or value. Accordingly, when the intensity or value of the reflected light is less than the predetermined intensity or value, the mobile device may determine that the contact state is not good, and output a notification message instructing the object to adjust the contact state. When it is determined that the contact state is good, the mobile device may continuously measure body fat.

For example, the mobile device may output a message instructing the user to accurately position his or her right hand on the electrodes.

According to an embodiment of the present invention, the mobile device may measure a pulse, an oxygen saturation (SpO2), and a tissue oxygen saturation (StO2) via an optical sensor. The mobile device may determine the contact state between the object and the electrodes, based on a pulse frequency measured via the optical sensor, when determining the contact state according to the impedance change cycle described above with reference to FIG. 12. The mobile device may provide the user with information about the measured tissue oxygen saturation together with body fat information.

According to an embodiment of the present invention, operations 1301 and 1303 may be performed before a body fat measurement. In other words, operations 1301 and 1303 may be performed before each operation of FIG. 8 is performed. In other words, the mobile device may check a contact state within a short period of time before measuring body fat, thereby preventing re-measurement due to an abnormal contact state.

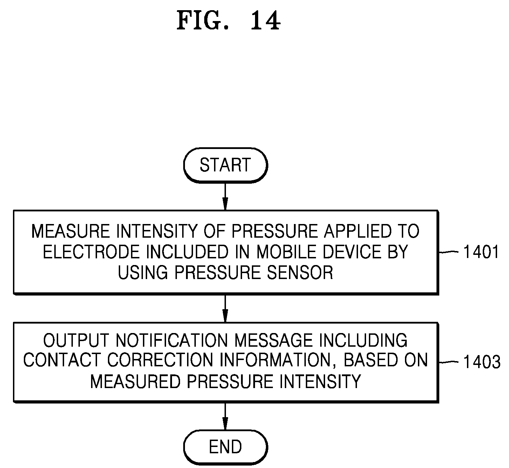

FIG. 14 is a flowchart of a method of checking a contact state based on a pressure intensity, according to an embodiment of the present invention.

In operation 1401, the mobile device may measure the intensity of a pressure applied to an electrode included in the mobile device by using a pressure sensor.

According to an embodiment of the present invention, when the electrode included in the mobile device is pressed with a predetermined intensity or more, the intensities and amounts of blood flowing on the first surface and the second surface of the object may decrease by at least a certain intensity and amount. When the intensities and amounts of blood flowing on the first and second surfaces of the object decrease by at least the certain intensity and amount, a measured pulse value and a measured impedance of the object may change. Accordingly, a pressure between the object and the electrode needs to be adjusted to perform accurate body fat measurement. Accordingly, the mobile device may measure the intensity of the pressure applied to the electrode by using a pressure sensor.

In operation 1403, the mobile device may output a notification message including contact correction information, based on the measured intensity of pressure.

According to an embodiment of the present invention, at least one of the first through fourth electrodes included in the mobile device may include a pressure sensor. Accordingly, the mobile device may measure the pressure applied to the electrode via the pressure sensor, and may output a notification message notifying the object to apply a certain pressure to the electrode, based on the measured pressure intensity. When it is determined that the contact state is good, the mobile device may continuously measure body fat.

According to an embodiment of the present invention, the pressure sensor may be positioned on a predetermined location within the mobile device instead of in the electrode. According to an embodiment of the present invention, the pressure sensor may include a strain gage, a piezoelectric element, and the like.

According to an embodiment of the present invention, the mobile device includes information about a reference pressure intensity based on a user input. When a pressure having the reference pressure intensity or greater is applied between the object and the electrode via the pressure sensor, the mobile device may output a notification message in order to reduce the intensity of pressure. For example, the mobile device may output to the user a message requesting the user to grab his or her left hand loosely.

According to an embodiment of the present invention, operations 1401 and 1403 may be performed before a body fat measurement. In other words, operations 1401 and 1403 may be performed before each operation of FIG. 8 is performed. In other words, the mobile device may check the contact state within a short period of time before measuring body fat, thereby preventing re-measurement due to an abnormal contact state.

According to an embodiment of the present invention, operations 1401 and 1403 may start before the body fat measurement and may continue during the body fat measurement. In other words, operations 1401 and 1403 may be performed together with each operation of FIG. 8.

Finally, according to an embodiment of the present invention, assuming that 20 seconds are required to measure impedance of the object, when the object maintains a good contact state for 10 seconds and a contact failure occurs at the moment when 10 seconds have elapsed, the mobile device may output a notification message, may measure impedance for only the other remaining 10 seconds after outputting the notification message, and may acquire impedance information based on a result of the measurement for 10 seconds before outputting the notification message and a result of the measurement for 10 seconds after outputting the notification message. The above-described methods may also be performed in this way.

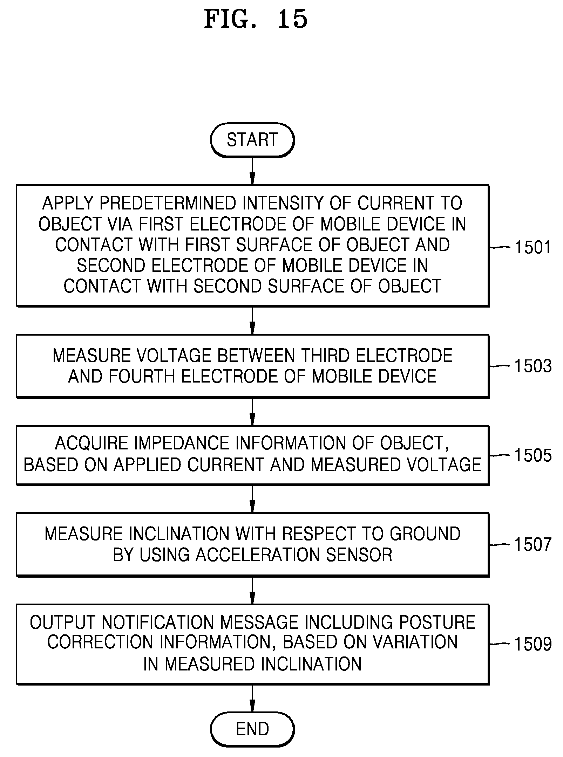

FIG. 15 is a flowchart of a method of determining whether a posture of an object needs to be corrected, based on an inclination variation, according to an embodiment of the present invention.

In operation 1507, the mobile device may measure an inclination with respect to the ground by using an acceleration sensor.

According to an embodiment of the present invention, the mobile device may include an acceleration sensor and may measure an inclination with respect to the ground by using the acceleration sensor. The mobile device may measure an inclination with respect to the ground by using an acceleration sensor and/or a gyroscope. A method of measuring an inclination with respect to the ground by using an acceleration sensor is obvious to one of ordinary skill in the art, and thus detailed descriptions thereof will be omitted.

In operation 1509, the mobile device may output a notification message including posture correction information, based on a variation in the measured inclination.

According to an embodiment of the present invention, impedance and body fat that are measured may be changed according to the posture of the object. In other words, impedance measured after the arms are stretched forward may be different from impedance measured after the arms are put down. Accordingly, when the inclination with respect to the ground changes by a predetermined size or greater during body fat measurement, the mobile device may output a notification message including posture correction information to the user.

For example, when the mobile device measured the inclination with respect to the ground to be 90° by using the acceleration sensor when starting measurement but the measured inclination changes by 10° or more during the measurement, the mobile device may instruct the user to put the arms up or down via a notification message so that the inclination between the mobile device and the ground is maintained at 90°. When a variation in the inclination is within a predetermined reference value, the mobile device may continue measuring body fat.

According to an embodiment of the present invention, operations 1501 and 1503 may start before a body fat measurement and may continue during the body fat measurement. In other words, operations 1501 and 1503 may be performed together with each operation of FIG. 8.

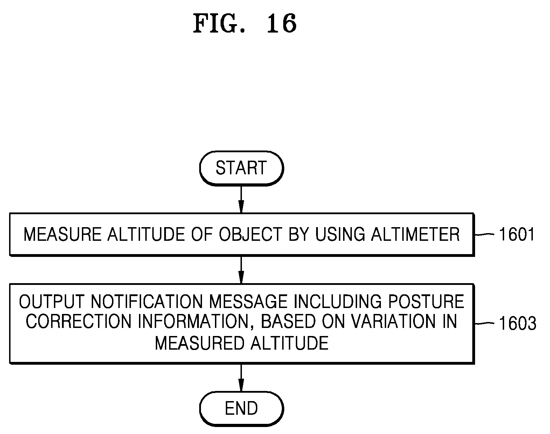

FIG. 16 is a flowchart of a method of determining whether a posture of an object needs to be corrected, based on an altitude variation, according to an embodiment of the present invention.

In operation 1601, the mobile device may measure an altitude by using an altimeter.

According to an embodiment of the present invention, the mobile device may include an altimeter, which may include an altitude sensor.

In operation 1603, the mobile device may output a notification message including posture correction information, based on a variation in the measured altitude.

As described above with reference to FIG. 15, impedance and body fat that are measured may be changed according to the posture of the object. In other words, impedance measured after the arms are stretched forward may be different from impedance measured after the arms are put down. Accordingly, when the measured altitude changes by a predetermined size or greater during body fat measurement, the mobile device may output a notification message including posture correction information to the object. When a variation in the altitude is less than or equal to a predetermined reference value, the mobile device may continue measuring body fat.

For example, the mobile device may measure an altitude from the ground to the mobile device before starting body fat measurement, and, when the altitude from the ground to the mobile device changes by a predetermined size or greater during the body fat measurement, the mobile device may output a notification message including posture correction information to the object.

Additionally, the mobile device may acquire body information of the object before starting the body fat measurement, and may inform the object of an altitude that is adequate for the body fat measurement, based on the acquired body information. For example, the mobile device may receive body information of the object including the height of the object, may calculate an altitude at the level of the shoulders of the object, and may output a notification message requesting the object to position the mobile device at the level of his or her shoulders.

According to an embodiment of the present invention, operations 1601 and 1603 may start before a body fat measurement and may continue during the body fat measurement. In other words, operations 1601 and 1603 may be performed together with each operation of FIG. 8.

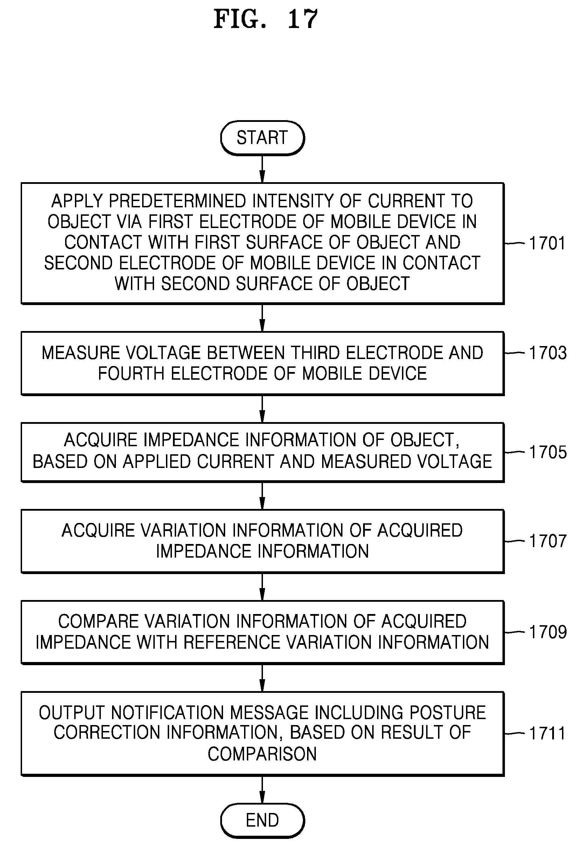

FIG. 17 is a flowchart of a method of determining whether a posture of an object needs to be corrected, based on a variation in impedance information, according to an embodiment of the present invention.

Operations 1701-1705 correspond to operations S801-S805 of FIG. 8, respectively, and thus detailed descriptions thereof will be omitted.

In operation 1707, the mobile device may acquire variation information of the impedance information acquired in operation 1705.

In operation 1709, the mobile device may compare the variation information of the impedance acquired in operation 1707 with reference variation information.

As described above, the impedance may continuously change according to various factors. The impedance may not only change according to a blood flow depending on heartbeat, but also may change when the object bends a joint, such as fingers, a wrist, or an elbow, or when an angle between the trunk and an arm of the object changes.

For example, a low impedance may be measured when a wrist joint or an arm joint is bent, compared with when the wrist joint or the arm joint is not bent, and a high impedance may be measured when the angle between the shoulder joint and the trunk of the object is equal to or greater than 90°, compared with when the angle between the shoulder joint and the trunk of the object is less than or equal to 90°. A low impedance may be measured when the muscle of the object stiffens by a predetermined amount or higher, compared with when the muscle of the object is relaxed.

According to an embodiment of the present invention, a variation in the impedance according to heartbeat may be less than or equal to 2 ohm per second. However, a variation in the impedance according to a motion of the object as described above may be equal to or greater than 2 ohm per second.

Accordingly, according to an embodiment of the present invention, the mobile device may acquire variation information of the measured impedance, may compare the acquired variation information with the reference variation information, and may determine that the object is highly likely to move, when the acquired variation information is higher than the reference variation information.

According to an embodiment of the present invention, the reference variation information may be reference information set by a user input.

In operation 1711, the mobile device may output a notification message including posture correction information, based on a result of the comparison.

For example, the mobile device may acquire a variation per unit time of the impedance measured in operation 1705, and, when the acquired variation per unit time is equal to or greater than 2 ohm/second, the mobile device may output a notification message requesting the object to maintain his or her posture to the object and may re-measure the impedance. The mobile device may also re-measure the impedance a predetermined time period after outputting the notification message.

When a total of 20 seconds are required to measure the impedance, the mobile device may acquire a variation in the impedance for 5 seconds, may determine whether the object is moving, based on the acquired impedance variation for 5 seconds, and may re-measure impedance or body fat in a short time.

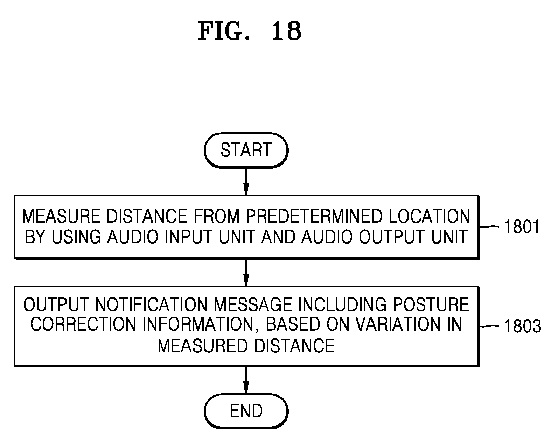

FIG. 18 is a flowchart of a method of determining whether a posture of an object needs to be corrected, based on a distance variation, according to an embodiment of the present invention.

In operation 1801, the mobile device may measure a distance from a predetermined location by using the audio input unit and the audio output unit.

For example, the mobile device may measure a distance from a surrounding geographical feature, such as a wall, the ground, or a ceiling. In other words, the mobile device may measure the distance from the surrounding geographical feature, by measuring a time period taken for a pulse generated by the audio output unit to return to the audio output unit.

According to an embodiment of the present invention, the audio input unit may include a microphone, and the audio output unit may include a speaker.

In operation 1803, the mobile device may output a notification message including posture correction information, based on a variation in the measured distance.

According to an embodiment of the present invention, when the object moves during a body fat measurement, lower or higher impedance than actual impedance of the object may be measured. Accordingly, the mobile device may ascertain a motion of the object during body fat measurement, and, when it is ascertained that the object moves, the mobile device may output a notification message requesting the object to maintain a standstill posture. When it is determined that the object maintain a standstill posture, the mobile device may continuously measure body fat.

According to an embodiment of the present invention, when the object moves, the distance from the surround geographical feature changes, and thus, when the distance from the surround geographical feature has changed by a predetermined reference value or more during body fat measurement, the mobile device may output a notification message including posture correction information.

For example, assuming that a distance from a wall measured by the mobile device by using the audio input unit and the audio output unit is 1 m, the mobile device may continuously measure the distance from the wall by using the audio input unit and the audio output unit during body fat measurement, and, when the distance from the wall changes by 10cm or more, the mobile device may output a notification message requesting the object to maintain a standstill state to the object.

In other words, according to an embodiment of the present invention, operations 1801 and 1803 may start before the body fat measurement and may continue during the body fat measurement. In other words, operations 1801 and 1803 may be performed together with each operation of FIG. 8.

Finally, according to an embodiment of the present invention, assuming that 20 seconds are required to measure impedance of the object, when the object maintains a standstill state for 10 seconds and then moves at the moment when 10 seconds elapse, the mobile device may output a notification message, may measure impedance for only the other remaining 10 seconds, and may acquire impedance information based on a result of the measurement for 10 seconds before outputting the notification message and a result of the measurement for 10 seconds after outputting the notification message. This may also apply to the above-described impedance measuring methods.

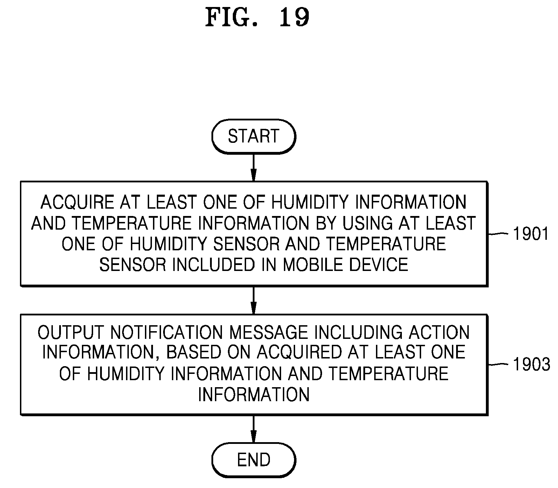

FIG. 19 is a flowchart of a method of determining whether an action of an object is necessary, based on at least one of humidity information and temperature information, according to another embodiment of the present invention.

In operation 1901, the mobile device may acquire at least one of humidity information and temperature information by using at least one of a humidity sensor and a temperature sensor included in the mobile device.

In operation 1903, the mobile device may output a notification message including action information, based on the acquired at least one of the humidity information and the temperature information.

For example, when a surrounding temperature is too high or a surrounding humidity is too low, the impedance of the object may not be accurately measured. When a skin state of the object is too dry, it may take long time to accurately measure the impedance.

Accordingly, the mobile device may acquire humidity information and temperature information of the surrounding environment or the object by using at least one of the humidity sensor and the temperature sensor included in the mobile device, and may analyze information about the surrounding environment. When the surrounding environment has a higher or lower temperature or humidity than a predetermined temperature or humidity, the mobile device may output a notification message notifying the object that the object needs to move from place to place or water, an emulsion, a toner, or the like needs to be applied to the first surface of the object.

According to an embodiment of the present invention, operations 1901 and 1903 may start before a body fat measurement and may continue during the body fat measurement. In other words, operations 1901 and 1903 may be performed together with each operation of FIG. 8.

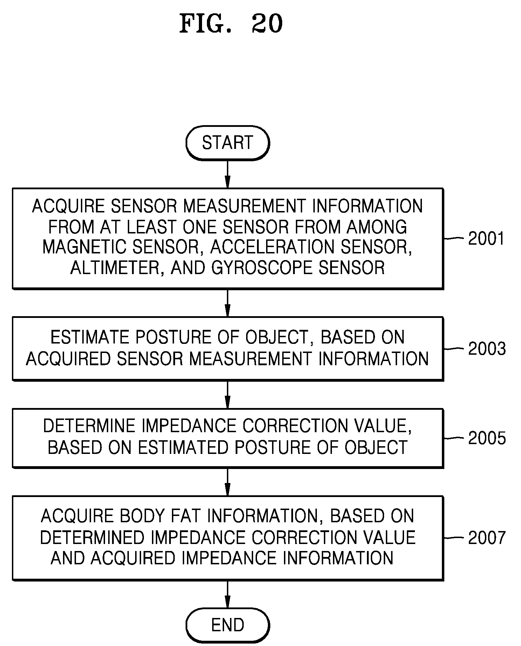

FIG. 20 is a flowchart of a method of determining an impedance correction value according to a posture of an object, according to an embodiment of the present invention.

In operation 2001, the mobile device may acquire sensor measurement information from at least one sensor.

According to an embodiment of the present invention, the at least one sensor may denote at least one of a magnetic sensor, an acceleration sensor, an altimeter, and a gyroscope sensor. According to an embodiment of the present invention, the sensor measurement information may include an angle between the mobile device and the ground or the object, an inclination therebetween, and an altitude therebetween.

In operation 2003, the mobile device may estimate a posture of the object, based on the acquired sensor measurement information.

According to an embodiment of the present invention, the mobile device may acquire location information of the mobile device, based on at least one of a magnetic sensor, an acceleration sensor, an altimeter, and a gyroscope sensor. According to an embodiment of the present invention, the location information is information about an altitude of the mobile device, an angle thereof, a coordinate thereof, a distance from a predetermined location, and the like, and may include information representing a location where the mobile device is positioned with respect to the object. The location information may also include information about whether the mobile device is on the level of the shoulders of the user or around the waist of the user.

According to an embodiment of the present invention, the mobile device may estimate the posture of the object by taking into account body information of the object together with the sensor measurement information. In other words, according to an embodiment of the present invention, the mobile device may estimate the posture of the object, based on the sensor measurement information.

For example, the mobile device may estimate the posture of the object according to whether the altitude measured via an altitude sensor is lower than the level of the shoulders of the user and whether a gravity acceleration of a z-axis component or a y-axis component is greater among the gravity accelerations of x-axis, y-axis, and z-axis components measured via the acceleration sensor or the gyroscope sensor. The acceleration sensor is obvious to one of ordinary skill in the art, and thus a detailed description thereof will be omitted.

According to an embodiment of the present invention, the mobile device may include an algorithm capable of estimating the posture of the object according to sensor measurement information of at least one acceleration sensor. For example, considering the height of the altimeter together with the height of the object, when the mobile device is distant from the ground by several cm or greater, or when the gravity acceleration in a z-axis gravity direction of the acceleration sensor is greater than the gravity acceleration in a y-axis gravity direction of the acceleration sensor, the mobile device may include an algorithm capable of estimating the posture of the object as a standard posture. The posture of the object will be further described later with reference to FIGS. 24 and 25.

The impedance of the object may also be measured by the mobile device displaying at least one measuring posture at which the impedance may be measured and by the object selecting a measuring posture from among the displayed at least one measuring posture and adopting the selected measuring posture.

In operation 2005, the mobile device may determine an impedance correction value, based on the estimated posture of the object.

As described above, the impedance measured by the mobile device may vary according to the posture of the user. For example, impedance that is about 50 Ω smaller may be measured with respect to a first posture of the object lowering his or her arms down his or her trunk and grabbing the mobile device than with respect to a standard posture of the object grabbing the mobile device at an angle of 90° to his or her trunk.

Accordingly, according to an embodiment of the present invention, the mobile device may apply a correction value depending on a posture to the impedance actually measured during body fat calculation, in order to accurately measure the body fat of the object.

In operation 2007, the mobile device may acquire body fat information, based on the determined impedance correction value and the acquired impedance information.

According to an embodiment of the present invention, the mobile device may acquire the impedance information based on the measured voltage and the applied current as described above with reference to FIG. 8, may determine the impedance correction value depending on the posture of the object, together with the impedance information as in operation 2005, and may acquire as accurate body fat information of the object as possible by applying the determined impedance correction value to the acquired impedance information.

According to an embodiment of the present invention, operations 2001 and 2003 may be performed before, during, and after a body fat measurement. In other words, operations 2001 and 2003 may be performed before, after, and during each operation of FIG. 8.

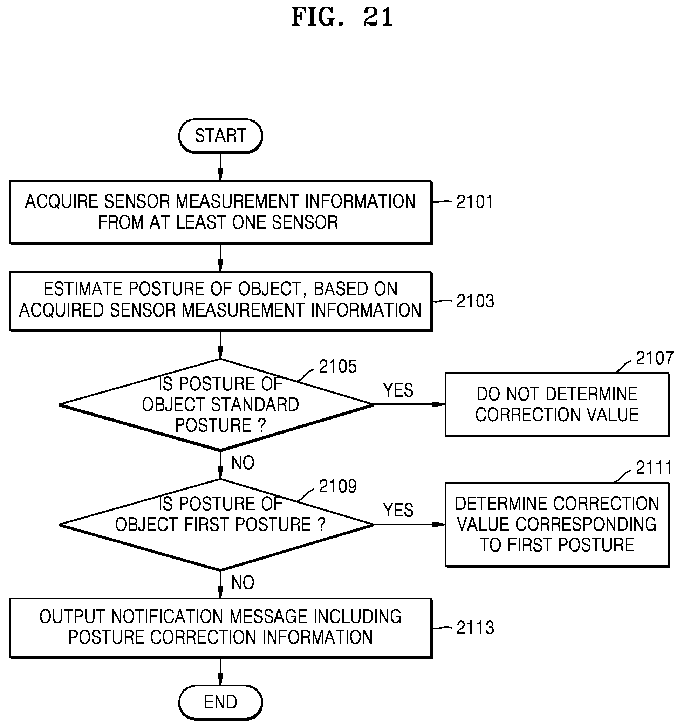

FIG. 21 is a detailed flowchart of a method of determining an impedance correction value according to a posture of an object, according to an embodiment of the present invention.

FIG. 21 is a detailed flowchart of FIG. 20, and may denote an algorithm included in the mobile device.

Operations 2101 and 2103 correspond to operations 2001 and 2003 of FIG. 20, respectively, and thus detailed descriptions thereof will be omitted.

In operation 2105, the mobile device may determine whether a posture of the object is a standard posture, based on the acquired sensor measurement information. For example, the mobile device may determine, as the standard posture, a posture of the object stretching his or her arms forward at an angle of 90° to his or her trunk. The mobile device may estimate a location of the mobile device and a posture of the object based on the acquired sensor measurement information and may estimate a posture currently taken by the object.

In operation 2105, when the object is taking the standard posture, additional correction of the impedance information may not be necessary. Accordingly, the mobile device may determine that an additional correction value does not need to be applied.

In operation 2109, when the mobile device estimates the posture of the object as a non-standard posture, the mobile device may determine whether the posture of the object is the first posture. According to an embodiment of the present invention, the first posture may be a posture of the object lowering his or her arms down his or her trunk and grabbing the mobile device. The standard posture and the first posture will be described in detail later with reference to FIGS. 24 and 25.

In operation 2111, when the mobile device estimates the posture of the object as the first posture, the mobile device may determine a correction value corresponding to the first posture.

In operation 2113, when the mobile device estimates the posture of the object as neither the standard posture nor the first posture, the mobile device may output a notification message including posture correction information. In other words, the mobile device may output a notification message requesting the object to take the standard posture or the first posture, and may also output a reference drawing, a reference image, or the like for informing the object of the standard posture and the first posture.

FIG. 22 is a flowchart of a method of determining an impedance correction value according to an internal temperature of the mobile device, according to an embodiment of the present invention.

In operation 2201, the mobile device may measure the internal temperature of the mobile device.

According to an embodiment of the present invention, the mobile device may include a sensor capable of measuring the internal temperature of the mobile device, and may measure the internal temperature of the mobile device via the sensor.

In operation 2203, the mobile device may determine an impedance correction value, based on the measured internal temperature.

According to an embodiment of the present invention, since many components, such as, a CPU, memory, and a battery, are operating within the mobile device and generate heat when operating, when the mobile device is used, the internal temperature of the mobile device may gradually increase. When the internal temperature of the mobile device increases, the impedance of the object may not be accurately measured. Thus, in order to accurately measure body fat and impedance of the object, the mobile device may acquire body fat information by applying an impedance correction value according to the internal temperature of the mobile device.

For example, as described above, the mobile device may use a reference resistor in order to accurately measure the impedance of the object. In other words, the mobile device may accurately measure the impedance of the object by comparing a voltage applied to the reference resistor with a voltage applied to between the third electrode and the fourth electrode. However, the size of the reference resistor may also change according to a temperature change. Accordingly, since the size of the reference resistor also changes according to the internal temperature of the mobile device, the mobile device may fail to accurately measure the impedance of the object. For example, based on the reference resistor, the voltage applied to the reference resistor, and the voltage applied to the object, the impedance of the object that is to be measured as 500 Ω may be measured as 480 Ω due to a change in the value of the reference resistor according to a temperature rise.

Accordingly, according to an embodiment of the present invention, the mobile device may measure the internal temperature of the mobile device and determine the impedance correction value according to a change in the internal temperature of the mobile device.

In operation 2205, the mobile device may acquire body fat information, based on the determined impedance correction value and the acquired impedance information.

According to an embodiment of the present invention, the mobile device may acquire the impedance information based on the measured voltage and the applied current as described above with reference to FIG. 8, may determine the impedance correction value depending on the internal temperature of the mobile device, together with the impedance information as in operation 2205, and may acquire as accurate body fat information of the object as possible by applying the determined impedance correction value to the acquired impedance information.

According to an embodiment of the present invention, operations 2201 and 2203 may be performed before, during, and after a body fat measurement. In other words, operations 2201 and 2203 may be performed before, after, and during each operation of FIG. 8.

According to an embodiment of the present invention, the methods of FIGS. 10-22 may be used independently or in various combinations.

FIG. 23 is a detailed block diagram of a structure of a mobile device according to an embodiment of the present invention.

The mobile device 100 according to an embodiment of the present invention may include the first electrode 101, the second electrode 102, the third electrode 103, the fourth electrode 104, the power unit 901, the voltage measurer 903, the controller 905, the output unit 907, a sensor unit 2310, a user input unit 2320, a communicator 2330, and a memory 2340.

The first electrode 101, the second electrode 102, the third electrode 103, the fourth electrode 104, the power unit 901, and the voltage measurer 903 correspond to those described above with reference to FIG. 9, and thus repeated descriptions thereof will be omitted.

According to an embodiment of the present invention, the controller 905 may measure and calculate the impedance of the object in order to acquire the impedance information of the object, and may change an analog signal corresponding to the measured impedance to a digital signal. Operations of the controller 905 correspond to those described above with reference to FIG. 9, and thus repeated descriptions thereof will be omitted.

According to an embodiment of the present invention, the controller 905 may include a posture estimator 915 and a correction value determiner 925.

According to an embodiment, the posture estimator 915 may estimate the posture of the object, based on sensor measurement information acquired by the sensor unit 2310. The posture estimator 915 may store an algorithm for estimating the posture of the object.

According to an embodiment of the present invention, the correction value determiner 925 may determine an impedance correction value, based on the estimated posture of the object. According to an embodiment of the present invention, the correction value determiner 925 may determine an impedance correction value according to the internal temperature of the mobile device.

According to an embodiment of the present invention, the controller 905 may control the output unit 907 to output a notification message.

According to an embodiment of the present invention, the output unit 907 may include a display 917, a motor 927, and an audio output unit 937.

According to an embodiment of the present invention, the display 917 displays a screen image including a moving picture, a text, an image, and the like, and may output various notification messages including posture correction information, action information, contact correction information, and information about the posture of the object.

According to an embodiment of the present invention, the motor 927 may generates a vibration of the mobile device, and the mobile device may output a notification message via the vibration. The mobile device may replace the motor 927 with another component that generates a vibration.

According to an embodiment of the present invention, the audio output unit 937 may include a speaker and may output a notification message via sound.

The output unit 907 may further include a light emission device, such as a lamp, and may output a notification message via the light emission device.

According to an embodiment of the present disclosure, the sensor 2310 may include a magnetic sensor 2311, an acceleration sensor 2312, an altimeter 2313, a thermometer 2314, a hygrometer 2315, an optical sensor 2316, and a pressure sensor 2317. The sensor 2310 may include only some of the enumerated plurality of sensors, or may further include a sensor in addition to the enumerated plurality of sensors.

The sensor unit 2310 may acquire information by using the sensors 2311-2317 under the control of the controller 905 and may provide the acquired information to the controller 905, and thus the controller 905 may check a contact state between the object and electrodes or the posture of the object and may determine the impedance correction value. Operations and acquired information of each sensor are obvious to one of ordinary skill in the art, and thus detailed descriptions thereof will be omitted.

According to an embodiment of the present invention, the user input unit 2320 may receive various pieces of information from a user. In other words, the user input unit 2320 may receive body information including the height, weight, gender, and the like of the user, and may also receive a command for starting body fat measurement from the user.

The user input unit 2320 may also include an audio input unit 2321. The audio input unit 2321 may include a microphone, and may receive a pulse generated by the audio output unit 937 and ascertain a distance from a predetermined location.

The user input unit 2320 may further include a camera, a touch pad, a touch screen, a keypad, a trackball, an electronic pen, a keyboard, a mouse, and the like, and embodiments are not limited thereto.

According to an embodiment of the present invention, the communicator 2330 may perform short-distance communication and long-distance communication, and, when some components shown in FIG. 9 or 23 are included and distributed in the cover or case of the mobile device and the main body of the mobile device except for the cover or case, the communicator 2330 may perform communication among the components.

According to an embodiment of the present invention, the memory 2340 may store all of the date, the time, user contact information, body information of the user, and so on.

According to an embodiment of the present invention, the mobile device 100 may further include a power amplifier, a switch, a band pass filter, an analog-to-digital converter (ADC), a multiplexer (MUX), and the like.

As described above, since a mobile device in the present specification also means the cover and case of the mobile device, some or all of the components of FIG. 9 or 23 may be included in the cover and case of the mobile device. Since the mobile device in the present specification may be, for example, a cellular phone, the power unit 901, the communicator 2330, the memory 2340, and the controller 905 may mean components included in the cellular phone.

FIGS. 24 and 25 illustrate postures of an object of which body fat is to be measured.

The posture of FIG. 24 may mean a posture of the object grabbing the mobile device 100 at an angle of 90° to the trunk of the object. In other words, the posture of FIG. 24 may mean a posture at which an angle between each shoulder joint and the trunk of the object is 90°. The posture of FIG. 25 may mean a posture of the object lowering his or her arms down the trunk of the object and grabbing the mobile device 100. In other words, the posture of FIG. 25 may mean a posture at which the mobile device is located around the waist of the object while the arms of the object are being straightened.

As described above with reference to FIGS. 20 and 21, the mobile device may acquire sensor measurement information from at least one sensor, may estimate a posture of the object based on the acquired sensor measurement information, and may determine an impedance correction value according to the posture of the object.

In other words, since the impedance of the object may be measured differently according to the posture of the object, the mobile device 100 may estimate whether the posture of the object is the posture of FIG. 24 or the posture of FIG. 25, may determine an impedance correction value corresponding to the estimated posture of the object, and may measure accurate body resistance by applying the determined impedance correction value to measured impedance information.

According to an embodiment of the present invention, the posture of FIG. 24 may be referred to as a standard posture, and the posture of FIG. 25 may be referred to as a first posture. The mobile device may also estimate a posture other than the postures of FIGS. 24 and 25, and may determine an impedance correction value corresponding to each measuring posture.

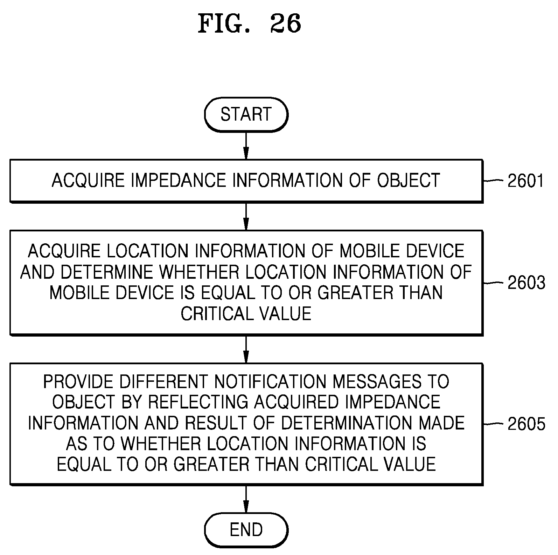

FIG. 26 is a flowchart of a method of providing information regarding a body fat measurement result of a mobile device, according to an embodiment of the present invention.

In operation 2601, the mobile device may acquire impedance information of an object.

According to an embodiment of the present invention, the mobile device may acquire the impedance information of the object, as described above with reference to FIGS. 1-25. In other words, the mobile device may acquire the impedance information of the object by measuring a current applied to the object and a voltage of the object. For example, the mobile device may acquire the impedance information of the object by using the first through fourth electrodes included in the mobile device or in the cover of the mobile device.

According to an embodiment of the present invention, the mobile device may acquire at least one of impedance information and body fat information.

In operation 2603, the mobile device may acquire location information of the mobile device and determine whether the location information of the mobile device is equal to or greater than a critical value.

According to an embodiment of the present invention, the location information of the mobile device is information about an altitude of the mobile device, an angle thereof, a coordinate thereof, a distance from a predetermined location, and the like, and may include information representing a location where the mobile device is positioned with respect to the object. The location information of the mobile device may also include information about whether the mobile device is on the level of the shoulders of a user, namely, the object, or is around the waist of the user.

According to an embodiment of the present invention, the mobile device may acquire the location information according to various methods. For example, the mobile device may measure an inclination between the mobile device and the ground by using an acceleration sensor included in the mobile device, may determine whether the measured inclination or a variation in the measured inclination is equal to or greater than a predetermined value to thereby acquire location information of the mobile device, and may determine whether the acquired location information of the mobile device is equal to or greater than a critical value. In other words, the mobile device may acquire information about where the mobile device is located.

According to an embodiment of the present invention, the mobile device may measure an altitude of the mobile device by using an altimeter included in the mobile device, may determine whether the measured altitude or a variation in the measured altitude is equal to or greater than a predetermined value to thereby acquire location information of the mobile device, and may determine whether the acquired location information of the mobile device is equal to or greater than a critical value.

According to an embodiment of the present invention, the mobile device may measure a distance from a predetermined location by using an audio input unit and an audio output unit included in the mobile device. In other words, the mobile device may measure a distance from a surrounding environment, such as a wall or ceiling around the object, and may determine whether a variation in the measured distance is equal to or greater than a predetermined reference value. For example, when a variation in the distance per second is equal to or greater than a critical value (predetermined reference value), the mobile device may output a notification message to the object.

According to an embodiment of the present invention, the location information of the mobile device may be determined based on sensor measurement information. The sensor measurement information may denote pieces of information measured using various sensors included in the mobile device, and the various sensors may include, but is not limited to, an acceleration sensor, a gyroscope sensor, an altimeter, a magnetic sensor, and so on. In other words, the above-described location information may be determined based on an inclination, an altitude, a distance, and the like measured based on the sensor measurement information.

According to an embodiment of the present invention, the critical value may denote a predetermined reference value or a predetermined value.

In operation 2605, the mobile device may provide different notification messages to the object by reflecting the acquired impedance information and a result of the determination made as to whether the location information is equal to or greater than the critical value.

According to an embodiment of the present invention, the different notification messages may denote notification messages determined based on a result of the determination, and the notification message may denote a notification message including the impedance information or body fat information depending on the impedance information or a notification message including posture correction information according to a result of the determination made as to whether the location information is equal to or greater than the critical value.

For example, the mobile device may estimate the posture of the object. The mobile device may acquire the location information of the mobile device based on the sensor measurement information, and may estimate a posture of the object based on the location information. In other words, the mobile device may estimate the posture of the object by determining whether the acquired location information is equal to or greater than the critical value. The mobile device may provide the object with at least one of a notification message including posture correction information corresponding to the estimated posture and a notification message including the impedance information or the body fat information.

The mobile device may provide a notification message including posture correction information to the object by reflecting corrected impedance information and a result of the determination made as to whether the location information is equal to or greater than the critical value. In other words, the mobile device may determine a location of the mobile device based on the location information and may provide a notification message based on a result of the determination.

According to an embodiment of the present invention, the mobile device may determine an impedance correction value, based on the estimated posture, and may acquire the corrected impedance information, based on the determined impedance correction value and the acquired impedance information. The mobile device may also provide a notification message including posture correction information, based on the corrected impedance information and the result of the determination made as to whether the location information is equal to or greater than the critical value. This corresponds to the description made with reference to FIGS. 1-25, and thus a repeated description thereof will be omitted.

According to an embodiment of the present invention, the mobile device may measure an internal temperature of the mobile device and determine the impedance correction value according to the measured internal temperature of the mobile device.

According to an embodiment of the present invention, the mobile device may acquire variation information of the impedance information, and may provide a notification message including posture correction information to the object, based on the acquired variation information and reference variation information.

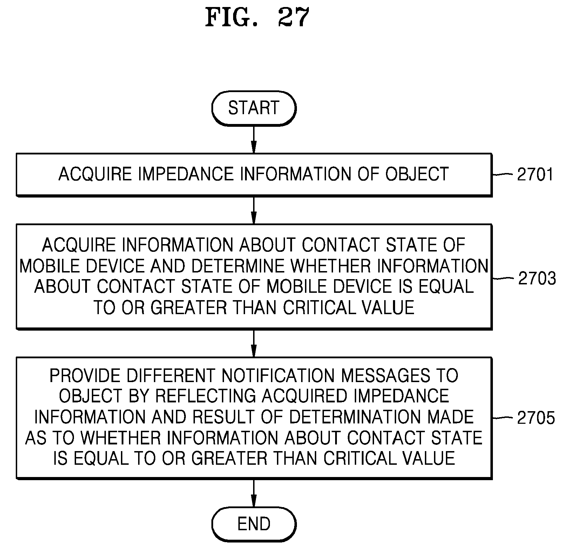

FIG. 27 is a flowchart of a method of providing information regarding a body fat measurement result of a mobile device, according to an embodiment of the present invention.

In operation 2701, the mobile device may acquire impedance information of an object.

According to an embodiment of the present invention, the mobile device may acquire the impedance information of the object, as described above with reference to FIGS. 1-25. In other words, the mobile device may acquire the impedance information of the object by measuring a current applied to the object and a voltage of the object. For example, the mobile device may acquire the impedance information of the object by using the first through fourth electrodes included in the mobile device or in the cover of the mobile device.

According to an embodiment of the present invention, the mobile device may acquire at least one of impedance information and body fat information.

In operation 2703, the mobile device may acquire information about a contact state of the mobile device and determine whether the information about the contact state of the mobile device is equal to or greater than a critical value.

According to an embodiment of the present invention, the information about the contact state of the mobile device is information about contact or non-contact between the mobile device and the object, a contact degree, a contact distance, a contact area, and the like, and may include, for example, information about the intensity of pressure applied to the mobile device or the electrodes included in the mobile device and a reflected light value of an optical sensor.

According to an embodiment of the present invention, the mobile device may acquire the information about the contact state according to various methods.

For example, the mobile device may measure the intensity of the pressure applied to the mobile device by using a pressure sensor included in the mobile device. The pressure sensor of the mobile device may be included in at least one of at least one electrode included in the mobile device. The mobile device may provide a notification message to the object by determining whether the measured pressure intensity or a variation in the pressure intensity is a predetermined reference value. This corresponds to the above-description, and thus a detailed description thereof will be omitted.

According to an embodiment of the present invention, the mobile device may measure a reflected light value of at least one of the first surface and the second surface of the object by using an optical sensor. The mobile device may determine whether the measured reflected light value or a variation in the reflected light value is equal to or greater than a predetermined value. For example, when the measured reflected light value is equal to or greater than the predetermined value, the mobile device may determine that the object and the mobile device are not close to each other within a predetermined distance. This corresponds to the above-description, and thus a detailed description thereof will be omitted.

According to an embodiment of the present invention, the mobile device may apply a current to the object by using the electrodes included in the mobile device, may measure the intensity of current received from the object via the electrodes of the mobile device, and may compare the measure current intensity with the intensity of current applied via the electrodes. By determining whether the measure current intensity is different from the intensity of current applied via the electrodes by a predetermined intensity or greater, the mobile device may determine whether the contact state is good, and may provide a notification message to the object. This corresponds to the above-description, and thus a detailed description thereof will be omitted.

According to an embodiment of the present invention, the critical value may denote a predetermined reference value or a predetermined value.

In operation 2705, the mobile device may provide different notification messages to the object by reflecting the acquired impedance information and a result of the determination made as to whether the location information is equal to or greater than the critical value.

According to an embodiment of the present invention, the different notification messages may denote notification messages determined based on a result of a determination, and the notification message may denote a notification message including the impedance information or body fat information depending on the impedance information or a notification message including contact correction information according to the result of the determination made as to whether the location information is equal to or greater than the critical value.

According to an embodiment of the present invention, the mobile device may measure an internal temperature, may determine an impedance correction value based on the measured internal temperature, and may acquire corrected impedance information, based on the determined impedance correction value and the acquired impedance information of the object. This corresponds to the description made with reference to FIGS. 1-25, and thus a repeated description thereof will be omitted.

The mobile device may provide a notification message including contact correction information to the object by reflecting the corrected impedance information and the result of the determination made as to whether the information about the contact state is equal to or greater than the critical value. In other words, the mobile device may determine a contact state between the mobile device and the object, based on the information about the contact state, and may provide a notification message including contact correction information based on a result of the determination.

According to an embodiment of the present invention, the mobile device may acquire not only the impedance information but also change cycle information of the impedance information, may compare the acquired change cycle information with reference change cycle information, and may provide a notification message including contact correction information to the object. As described above, by comparing the change cycle information of the impedance information with the reference change cycle information, the mobile device may determine the contact state between the object and the mobile device, and thus may provide a notification message based on a result of the determination.

The mobile device may also acquire at least one of humidity information and temperature information by using a humidity sensor and a temperature sensor included in the mobile device, and may provide a notification message including action information to the object, based on the acquired at least one of the humidity information and the temperature information. This is the same as the description made with reference to FIGS. 1-25, and thus a repeated description thereof will be omitted.

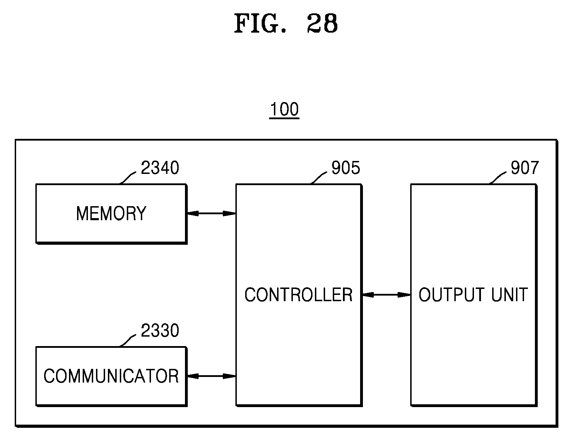

FIG. 28 is a block diagram of a mobile device according to an embodiment of the present invention.

A mobile device 100 according to an embodiment of the present invention may include the controller 950, the output unit 907, a communicator 2330, and a memory 2340.

According to an embodiment of the present invention, the controller 905 may acquire impedance information of an object and location information of the mobile device and may determine whether the location information of the mobile device is equal to or greater than a critical value. The controller 905 may acquire information about a contact state and may determine whether the acquired information about the contact state is equal to or greater than the critical value.

According to an embodiment of the present invention, the above-described determinations may mean determining where the mobile device is located and whether the contact state of the mobile device is good, by comparing the acquired location and the acquired information about the contact state with predetermined reference values, respectively.

According to an embodiment of the present invention, the output unit 907 may provide different notification messages to the object by reflecting the acquired impedance information and the results of the determinations made as to whether the acquired information is equal to or greater than the critical value. In other words, the output unit 907 may provide a notification message including the impedance information to the object, or may provide to the object a notification message including posture correction information or contact correction information based on the results of the determinations.

According to an embodiment of the present invention, the output unit 907 may provide measured body fat information based on the impedance information.

According to an embodiment of the present invention, the mobile device 100 may further include a sensor unit (not shown).

According to an embodiment of the present invention, the mobile device 100 may measure an inclination with respect to the ground by using an acceleration sensor, and the controller 905 may determine whether the measured inclination or a variation in the measured inclination is equal to or greater than a predetermined value.

According to an embodiment of the present invention, the mobile device 100 may measure an altitude of the mobile device by using an altimeter, and the controller 905 may determine whether the measured inclination or a variation in the measured inclination is equal to or greater than a predetermined value.

According to an embodiment of the present invention, the mobile device 100 may measure the intensity of pressure applied to the mobile device by using a pressure sensor, and the controller 905 may determine whether the measured pressure intensity or a variation in the measured pressure intensity is equal to or greater than a predetermined value.

According to an embodiment of the present invention, the mobile device 100 may measure a reflected light value of at least one of the first and second surfaces of the object by using an optical sensor, and the controller 905 may determine whether the measured reflected light value or a variation in the reflected light value is equal to or greater than a predetermined value.

The mobile device may also acquire humidity information and temperature information by using a humidity sensor and a temperature sensor included in the mobile device, and the output unit 907 may provide a notification message including action information to the object by reflecting at least one of the humidity information and the temperature information, the impedance information, and the results of the determinations made as to whether the acquired information is equal to or greater than the critical value.

According to an embodiment of the present invention, the mobile device 100 may further include an audio input unit and an audio output unit, and the controller 905 may measure a distance from a predetermined location by using the audio input unit and the audio output unit and may determine whether a variation in the measured distance is equal to or greater than a predetermined value.

According to an embodiment of the present invention, the mobile device 100 may apply a current to the body of the object by using electrodes included in the mobile device, may measure the intensity of a current received from the object, and may compare 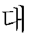 the measured current intensity with the intensity of the applied current to thereby determine whether a difference between the two current intensities is equal to or greater than a predetermined value. Based on a result of the determination regarding the difference between current intensities, the mobile device may provide a notification message including contact correction information to the object.

According to an embodiment of the present invention, the controller 905 may further include a posture estimator and a correction value determiner. The posture estimator may estimate a posture of the object, based on the location information of the mobile device, and the correction value determiner may determine an impedance correction value, based on the estimated posture of the object, an internal temperature, and the like. This corresponds to the above-description, and thus a detailed description thereof will be omitted.

According to an embodiment of the present invention, the mobile device 100 may include the communicator 2330 and the memory 2340, and the communicator 2330 and the memory 2340 correspond to those described above with reference to FIG. 23. Thus, detailed descriptions thereof will be omitted.

An apparatus according to the present invention may comprise a processor, a memory for storing program data and executing it, a permanent storage unit such as a disk drive, a communications port for handling communications with external devices, and user interface devices, including a touch panel, keys, buttons, etc. When software modules or algorithms are involved, these software modules may be stored as program instructions or computer readable codes executable on a processor on a computer-readable recording medium. Examples of the computer-readable recording medium include magnetic storage media (e.g., read-only memory (ROM), random-access memory (RAM), floppy disks, hard disks, etc.), and optical recording media (e.g., CD-ROMs, or Digital Versatile Discs (DVDs)). The computer-readable recording medium can also be distributed over network coupled computer systems so that the computer readable code is stored and executed in a distributive manner. This media can be read by the computer, stored in the memory, and executed by the processor.

All references, including publications, patent applications, and patents, cited herein are hereby incorporated by reference to the same extent as if each reference were individually and specifically indicated to be incorporated by reference and were set forth in its entirety herein.

To promote understanding of one or more exemplary embodiments, reference has been made to the exemplary embodiments illustrated in the drawings, and specific language has been used to describe these embodiments. However, no limitation of the scope of the inventive concept is intended by this specific language, and exemplary embodiments should be construed to encompass all exemplary embodiments that would normally occur to one of ordinary skill in the art.

Embodiments may be described in terms of functional block components and various processing steps. Such functional blocks may be realized by any number of hardware and/or software components configured to perform the specified functions. For example, embodiments may employ various integrated circuit (IC) components, e.g., memory elements, processing elements, logic elements, look-up tables, and the like, which may carry out a variety of functions under the control of one or more microprocessors or other control devices. Similarly, where the elements are implemented using software programming or software elements, the embodiments described herein may be implemented with any programming or scripting language such as C, C++, Java, assembler language, or the like, with the various algorithms being implemented with any combination of data structures, objects, processes, routines or other programming elements. Functional aspects may be implemented in algorithms that are executed on one or more processors. Furthermore, the embodiments described herein could employ any number of conventional techniques for electronics configuration, signal processing and/or control, data processing and the like. The words "mechanism", "element", "means", and "configuration" are used broadly and are not limited to mechanical or physical embodiments, but can include software routines in conjunction with processors, etc.

The particular implementations shown and described herein are illustrative embodiments and are not intended to otherwise limit the scope of embodiments in any way. For the sake of brevity, conventional electronics, control systems, software development and other functional aspects of the systems may not be described in detail. Furthermore, the connecting lines, or connectors shown in the various figures presented are intended to represent exemplary functional relationships and/or physical or logical couplings between the various elements. It should be noted that many alternative or additional functional relationships, physical connections or logical connections may be present in a practical apparatus. Moreover, no item or component is essential to the practice of the inventive concept unless the element is specifically described as "essential" or "critical".

The use of the terms "a" and "an" and "the" and similar referents in the context of describing the present invention (especially in the context of the following claims) are to be construed to cover both the singular and the plural. Furthermore, recitation of ranges of values herein are merely intended to serve as a shorthand method of referring individually to each separate value falling within the range, unless otherwise indicated herein, and each separate value is incorporated into the specification as if it were individually recited herein. Also, the steps of all methods described herein can be performed in any suitable order unless otherwise indicated herein or otherwise clearly contradicted by context. Embodiments of the present invention are not limited to the described order of the operations. The use of any and all examples, or exemplary language (e.g., "such as") provided herein, is intended merely to better illuminate the inventive concept and does not pose a limitation on the scope of the inventive concept unless otherwise claimed. Numerous modifications and adaptations will be readily apparent to one of ordinary skill in the art without departing from the spirit and scope.

While the present invention has been particularly shown and described with reference to exemplary embodiments thereof, it will be understood that various changes in form and details may be made therein without departing from the spirit and scope of the following claims.

## Claims

1. A method of providing information about a result of measuring body fat by a mobile device, the method comprising:
acquiring impedance information of an object, wherein the acquiring is performed by the mobile device;
acquiring location information of the mobile device and determining whether the location information of the mobile device is equal to or greater than a critical value; and
providing different notification messages to the object based on the impedance information and a result of the determining of whether the location information of the mobile device is equal to or greater than the critical value.

2. The method of claim 1, wherein the acquiring of the location information of the mobile device and the determining of whether the location information of the mobile device is equal to or greater than the critical value comprises:
measuring an inclination between the mobile device and ground by using an acceleration sensor of the mobile device; and
determining whether the measured inclination or a variation in the measured inclination is equal to or greater than a predetermined value.

3. The method of claim 1, wherein the acquiring of the location information of the mobile device and the determining of whether the location information of the mobile device is equal to or greater than the critical value comprises:
measuring an altitude of the mobile device by using an altimeter of the mobile device; and
determining whether the measured altitude or a variation in the measured altitude is equal to or greater than a predetermined value.

4. The method of claim 1, wherein the acquiring of the location information of the mobile device and the determining of whether the location information of the mobile device is equal to or greater than the critical value comprises:
measuring a distance from a predetermined location by using an audio input unit and an audio output unit included in the mobile device; and
determining whether a variation in the measured distance is equal to or greater than a predetermined reference value.

5. The method of claim 1, wherein
the location information of the mobile device is determined based on sensor measurement information,
the method further comprising:
estimating a posture of the object;
determining an impedance correction value, based on the estimated posture; and
acquiring corrected impedance information based on the acquired impedance information and the impedance correction value, and
the providing of the different notification messages to the object based on the impedance information and the result of the determining of whether the location information of the mobile device is equal to or greater than the critical value comprises providing a notification message including posture correction information to the object, based on the corrected impedance information and the result of the determining of whether the location information of the mobile device is equal to or greater than the critical value.

6. The method of claim 1, further comprising:
measuring an internal temperature of the mobile device;
determining an impedance correction value, based on the measured internal temperature; and
acquiring corrected impedance information based on the acquired impedance information and the impedance correction value,
wherein the providing of the different notification messages to the object based on the impedance information and the result of the determining of whether the location information of the mobile device is equal to or greater than the critical value comprises providing a notification message including posture correction information to the object, based on the corrected impedance information and the result of the determining of whether the location information of the mobile device is equal to or greater than the critical value.

7. The method of claim 1, wherein
the acquiring of the impedance information comprises:
acquiring variation information of the impedance information; and
comparing the variation information of the impedance information with reference variation information,
the providing of the different notification messages to the object based on the impedance information and the result of the determining of whether the location information of the mobile device is equal to or greater than the critical value comprises providing a notification message including posture correction information to the object, based on a result of the comparing and the result of the determining of whether the location information of the mobile device is equal to or greater than the critical value.

8. A method of providing information about a result of measuring body fat by a mobile device, the method comprising:
acquiring impedance information of an object, wherein the acquiring is performed by the mobile device;
acquiring information about a contact state of the mobile device and determining whether the information about the contact state of the mobile device is equal to or greater than a critical value; and
providing different notification messages to the object based on the information about the contact state of the mobile device and a result of the determining of whether the information about the contact state of the mobile device is equal to or greater than the critical value.

9. The method of claim 8, wherein the acquiring of the information about the contact state of the mobile device and the determining of whether the information about the contact state of the mobile device is equal to or greater than the critical value comprises:
measuring an intensity of pressure applied to the mobile device by using a pressure sensor of the mobile device; and
determining whether the measured pressure intensity or a variation in the measured pressure intensity is equal to or greater than a predetermined reference value.

10. The method of claim 8, wherein the acquiring of the information about the contact state of the mobile device and the determining of whether the information about the contact state of the mobile device is equal to or greater than the critical value comprises:
measuring a reflected light value of at least one of a first surface and a second surface of the object by using an optical sensor of the mobile device; and
determining whether the measured reflected light value or a variation in the measured reflected light value is equal to or greater than a predetermined value.

11. The method of claim 8, wherein the acquiring of the information about the contact state of the mobile device and the determining of whether the information about the contact state of the mobile device is equal to or greater than the critical value comprises:
applying a current to the object by using an electrode included in the mobile device;
measuring an intensity of current received from the object via the electrode included in the mobile device;
comparing the measured intensity of the current received from the object with the intensity of the current applied to the object via the electrode; and
determining whether a difference between the two current intensities is equal to or greater than a predetermined value.

12. The method of claim 8, further comprising:
measuring an internal temperature of the mobile device;
determining an impedance correction value, based on the measured internal temperature; and
acquiring corrected impedance information based on the acquired impedance information and the impedance correction value,
wherein the providing of the different notification messages to the object based on the impedance information and the result of the determining of whether the information about the contact state of the mobile device is equal to or greater than the critical value comprises providing a notification message including contact correction information to the object, based on the corrected impedance information and the result of the determining of whether the information about the contact state of the mobile device is equal to or greater than the critical value.

13. The method of claim 8, wherein
the acquiring of the impedance information comprises:
acquiring change cycle information of the impedance information; and
comparing the change cycle information of the impedance information with reference change cycle information, and
the providing of the different notification messages to the object based on the impedance information and the result of the determining of whether the information about the contact state of the mobile device is equal to or greater than the critical value comprises providing a notification message including contact correction information to the object, based on a result of the comparison and the result of the determining of whether the information about the contact state of the mobile device is equal to or greater than the critical value.

14. The method of claim 8, further comprising acquiring at least one of humidity information and temperature information by using at least one of a humidity sensor and a temperature sensor included in the mobile device,
wherein the providing of the different notification messages to the object based on the impedance information and the result of the determining of whether the information about the contact state of the mobile device is equal to or greater than the critical value comprises providing a notification message including action information to the object, by reflecting the at least one of the humidity information and the temperature information, the impedance information, and the result of the determining of whether the information about the contact state of the mobile device is equal to or greater than the critical value.

15. A mobile device comprising:
a controller configured to acquire impedance information of an object, acquire location information of the mobile device, and determine whether the location information of the mobile device is equal to or greater than a critical value; and
an output unit configured to provide different notification messages to the object based on the impedance information and a result of the determining of whether the location information of the mobile device is equal to or greater than the critical value.

16. The mobile device of claim 15, further comprising a sensor unit configured to measure an inclination between the mobile device and ground by using an acceleration sensor of the mobile device,
wherein the controller determines whether the inclination measured by the sensor unit or a variation in the measured inclination is equal to or greater than a predetermined value.

17. The mobile device of claim 15, further comprising a sensor unit configured to measure an altitude of the mobile device by using an altimeter of the mobile device,
wherein the controller determines whether the altitude measured by the sensor unit or a variation in the measured altitude is equal to or greater than a predetermined value.

18. The mobile device of claim 15, further comprising an audio input unit and an audio output unit,
wherein the controller measures a distance from a predetermined location by using the audio input unit and the audio output unit and determines whether a variation in the measured distance is equal to or greater than a predetermined value.

19. The mobile device of claim 15, further comprising a sensor unit configured to acquire sensor measurement information, wherein the location information of the mobile device is determined based on the sensor measurement information,
wherein
the controller comprises: a posture estimator configured to estimate a posture of the object, based on the acquired location information of the mobile device; and a correction value determiner configured to determine an impedance correction value based on the estimated posture, and acquire corrected impedance information based on the acquired impedance information and the determined impedance correction value, and
the output unit provides a notification message including posture correction information to the object, based on the corrected impedance information and a result of the determining of whether the location information of the mobile device is equal to or greater than the critical value.

20. The mobile device of claim 15, further comprising a sensor unit configured to measure an internal temperature of the mobile device,
wherein
the controller further comprises a correction value determiner configured to determine an impedance correction value based on the measured internal temperature,
the controller acquires corrected impedance information based on the acquired impedance information and the determined impedance correction value, and
the output unit provides a notification message including posture correction information to the object, based on the corrected impedance information and a result of the determining of whether the location information of the mobile device is equal to or greater than the critical value.

21. The mobile device of claim 15, wherein
the controller acquires variation information of the impedance information and compares the acquired variation information of the impedance information with reference variation information, and
the output unit provides a notification message including posture correction information to the object, based ona result of the comparison and the result of the determining of whether the location information of the mobile device is equal to or greater than the critical value.

22. A mobile device comprising:
a controller configured to acquire impedance information of an object, acquire information about a contact state of the mobile device, and determine whether the information about the contact state of the mobile device is equal to or greater than a critical value; and
an output unit configured to provide different notification messages to the object based on the impedance information and a result of the determining of whether the information about the contact state of the mobile device is equal to or greater than the critical value.

23. The mobile device of claim 22, further comprising a sensor unit configured to measure an intensity of pressure applied to the mobile device by using a pressure sensor of the mobile device,
wherein the controller determines whether the pressure intensity measured by the sensor unit or a variation in the measured pressure intensity is equal to or greater than a predetermined value.

24. The mobile device of claim 22, further comprising a sensor unit configured to measure a reflected light value of at least one of a first surface and a second surface of the object by using an optical sensor of the mobile device,
wherein the controller determines whether the reflected light value measured by the sensor unit or a variation in the measured reflected light value is equal to or greater than a predetermined value.

25. The mobile device of claim 22, wherein the controller applies a current to a body of the object by using an electrode included in the mobile device, measures an intensity of a current received from the object, compares the measured intensity of the current received from the object with the intensity of the current applied via the electrode, and determines whether a difference between the two current intensities is equal to or greater than a predetermined value.

26. The mobile device of claim 22, further comprising a sensor unit configured to measure an internal temperature of the mobile device,
wherein the controller further comprises a correction value determiner configured to determine an impedance correction value based on the measured internal temperature,
the controller acquires corrected impedance information based on the acquired impedance information and the determined impedance correction value, and
the output unit provides a notification message including contact correction information to the object, based on the corrected impedance information and a result of the determining of whether the information about the contact state of the mobile device is equal to or greater than the critical value.

27. The mobile device of claim 22, wherein
the controller acquires change cycle information of the impedance information and compares the acquired change cycle information of the impedance information with reference change cycle information, and
the output unit provides a notification message including contact correction information to the object, based on a result of the comparing and the result of the determining of whether the information about the contact state of the mobile device is equal to or greater than the critical value.

28. The mobile device of claim 22, further comprising a sensor unit configured to acquire at least one of humidity information and temperature information by using at least one of a humidity sensor and a temperature sensor of the mobile device,
wherein the output unit provides a notification message including action information to the object, based on the at least one of the humidity information and the temperature information, the impedance information, and the result of the determining of whether the information about the contact state of the mobile device is equal to or greater than the critical value.

29. A non-transitory computer-readable recording medium having recorded thereon a computer program, which, when executed by a computer, performs the method of one of claims 1-14.
